# EUROPEAN PATENT SPECIFICATION

(11) **EP 0 629 133 B1**
(45) Date of publication and mention of the grant of the patent: **29.11.2000**
(21) Application number: 93902846.0
(22) Date of filing: 31.12.1992
(51) Int. Cl.: A61K 51/08, C07K 7/08

(54) **PEPTIDE-METAL ION PHARMACEUTICAL APPLICATIONS**
PHARMAZEUTISCHE ANWENDUNGEN AUF DER BASIS VON PEPTID-METALL-IONEN
APPLICATIONS PHARMACEUTIQUES A BASE D'IONS METALLIQUES ET PEPTIDES

(30) Priority: 03.01.1992 US 816476; 03.01.1992 US 816477; 20.02.1992 US 840077; 30.12.1992 US 998820; 30.12.1992 US 998910
(43) Date of publication of application: 21.12.1994
(73) Proprietor: Rhomed, Incorporated, Albuquerque, New Mexico 87109 (US)
(72) Inventor: RHODES, Buck, A., Albuquerque, NM 87106 (US); ZAMORA, Paul, O., Albuquerque, NM 87106 (US)
(74) Representative: Braun, André
(86) International application number: US9211334
(87) International publication number: WO9312819

(56) References cited:
- EP-A- 0 196 669
- EP-A- 0 210 684
- EP-A- 0 250 013
- EP-A- 0 359 347
- WO-A-88/07382
- WO-A-89/10760
- WO-A-90/15818
- WO-A-91/01144
- WO-A-91/17173
- WO-A-92/13572
- CA-A- 2 016 235
- GB-A- 2 225 579
- US-A- 4 917 878
- US-A- 5 102 990
- US-A- 5 116 596
- US-A- 5 128 119
- US-A- 5 277 892
- US-A- 5 277 893
- 39TH ANNUAL MEETING OF THE SOCIETY OF NUCLEAR MEDICINE, LOS ANGELES, CALIFORNIA, USA, JUNE 9-12, 1992. & J NUCL MED, VOL. 33, NO. 5 (SUPPL.). 1992. PAGES 1029-1030, ABSTRACT 878 ZAMORA P O et al 'DIRECT TECHNETIUM-99M LABELING OF A PLATELET-BINDING LAMININ PEPTIDE'
- 39TH ANNUAL MEETING OF THE SOCIETY OF NUCLEAR MEDICINE, LOS ANGELES, CALIFORNIA, USA, JUNE 9-12, 1992. & J NUCL MED, VOL. 33, NO. 5 (SUPPL.). 1992, PAGES 1029-1030, ABSTRACT 869 M. NAKAYAMA et al. 'DIRECT Tc-99m LABELING OF ANTIBODY WITH MACROMOLECULAR Sn(II) COMPLEXES'
- 39TH ANNUAL MEETING OF THE SOCIETY OF NUCLEAR MEDICINE, LOS ANGELES, CALIFORNIA, USA, JUNE 9-12, 1992. & J NUCL MED, VOL. 33, NO. 5 (SUPPL.), 1992, PAGES 1029-1030, ABSTRACT 868 ZAMORA P O et al 'A SECOND Tc-99m BINDING SITE IN IgG IS RELATED TO HISTIDINE GROUPS'
- UNDERSTANDING BREAST CANCER: CLINICAL AND LABORATORY CONCEPTS. RICH MA, HAGER JC, FURMANSKI P, EDS. NEW YORK, MARCEL DEKKER, INC., PAGES 87-96, 1983. Liotta LA et al 'LAMININ RECEPTORS ON HUMAN BREAST CARCINOMA: ROLE IN INVASION OF THE EXTRACELLULAR MATRIX'
- PROC ANNU MEET AM ASSOC CANCER RES, VOL. 30, ABSTRACT 390, 1989 Sandrock D et al 'KINETICS OF IV-INJECTED 131I-LABELED LAMININ-C1 FRAGMENT IN NORMAL AND TUMOR-BEARING NUDE MICE (MEETING ABSTRACT)'
- BIOCHIM BIOPHYS ACTA, VOL. 1182, NO. 2, PAGE(S) 197-204, 1993. ZAMORA P O et al 'BIOLOGICAL DISTRIBUTION OF TECHNETIUM-99M LABELED YIGSR AND IKVAV LAMININ PEPTIDES IN RODENTS TECHNETIUM-99M IKVAV PEPTIDE LOCALIZES TO THE LUNG.'
- 40TH ANNUAL MEETING OF THE SOCIETY OF NUCLEAR MEDICINE, TORONTO, ONTARIO, CANADA, JUNE 8-11, 1993.;& J NUCL MED,, VOL. 34, NO. 5 SUPPL., PAGE(S) 244P, 1993. ZAMORA P O et al 'LUNG UPTAKE OF TECHNETIUM-99M LAMININ PEPTIDE PA22-2 IS DECREASED IN EMPHYSEMA AND INCREASED IN TUMORED LUNG'
- 41ST ANNUAL MEETING OF THE SOCIETY OF NUCLEAR MEDICINE, ORLANDO, FLORIDA, USA, JUNE 5-8, 1994.;& JOURNAL OF NUCLEAR MEDICINE 35, NO. 5 SUPPL., PAGE(S) 106P, 1994. Wang G-J et al 'A Tc-99m labeled laminin derived peptide, Tc-99m-YIGSR for thrombus specific imaging.'
- INTERNATIONAL JOURNAL OF PHARMACEUTICS, vol.79, 12 January 1992, AMSTERDAM, NL pages 77 - 80 M.V. PIMM ET AL '111-In labelling of a branched polypeptide drug carrier with a poly(L-lysine) backbone'
- 7th International Symposium on Radiopharmacology, Abstracts, (1991) COX et al., "Technetium Labelled Somato statin a Potential Agent for In Vivo Tumor Localization", p. 16.
- Immunology (1991), Volume 72, THOMPSON et al., "Identification of an Amino Acid Sequence in the Iamminan A Chain Mediating Mask Cell Attachment and Spreading", pp. 144-149.

## Description

This invention relates to a method of labelling peptides with radioactive, paramagnetic and other medically useful metal ions, and compositions comprising such labelled peptides for the detection of thrombus, cancer, infection, inflammation and various lung diseases, pathologies and abnormalities.

### Background

The use of proteins, particularly antibodies, as biologically active targeting agents for medically useful metal ions has been explored. These products can be administered to the human body to visualise or monitor functioning of various parts of the body or to determine the presence and location of particular antigens, antibodies, hormones or the like; and can be used in the treatment of various disease states. Antibodies and antibody fragments have been labelled with a number of radionucleides for use in clinical diagnosis. Radionucleides commonly used include ¹³¹I, ¹²⁵I, ¹²³I, ^{99m}Tc, ⁶⁷Ga, and ¹¹¹In for diagnostic imaging; and radionucleides such as ⁹⁰Y, ¹⁸⁸Re, and ¹⁸⁶Re, and to a lesser extent, ¹⁹⁹Au, ¹³¹I and ⁶⁷Cu for targeted therapy, primarily in the treatment of cancer. There are also useful metals for magnetic resonance imaging, including gadolinium, manganese, copper, iron, gold and europium, which are not radioisotopes. So far, limited work have been done with labelling with positron-emitting radiometals, although some types of proteins, such as transferrin and human serum albumin, have been labelled with ⁶⁸Ga.

### Antibody Labelling

Two primary methods have been employed to label antibodies with radiometals, with particular emphasis having been placed on radiolabelling with ^{99m}Tc. In one method, bifunctional chelates are conjugated to the antibody, and the bifunctional chelate is then radiolabelled. A variety of bifunctional chelates have been employed; most involve metal ion binding to thiolate groups, and may also involve metal ion binding to amide, amine or carboxylate groups. Representative bifunctional chelates include ethylenediamine tetraacetic acid (EDTA), diethylenetetramine-pentaacetic acid (DTPA), chelates of diamide-dimercaptides (N₂S₂), and variations on the foregoing, such as chelating compounds incorporating N₂S₃, N₂S₄ or N₃S₃ metal binding sites, and metallothionine. The alternative method of radiolabeling antibodies involves reduction of disulfide bonds in the protein, with subsequent binding of the metal ion to thiolate groups. A variety of reducing agents have been employed, including stannous salts, dithiothreitol and 2 -mercaptoethanol.

Antibodies and antibody fragments have been labeled with a number of radionuclides for use in clinical diagnosis. These radionuclides include ¹³¹I, ¹²⁵I, ¹²³I, ^{99m}Tc, ⁶⁷Ga, and ¹¹¹In. So far, only ^{99m}Tc and ¹¹¹In-labeled antibody preparations are widely used in clinical settings. For diagnostic imaging, both isotopes should be ideal; however, clinical limitations, including affinities for liver and kidneys that limit detection of abdominal diseases, have prompted searches for other imaging radionuclides.

Antibodies have also been labeled with a variety of radionuclides for potential use in targeted immunotherapy (Pietersz GA, Kannellos J, Smyth MJ, *et al*. The use of monoclonal antibody conjugates for the diagnosis and treatment of cancer. *Immunol Cell Biol* 65:111-125, 1987). These radionuclides include ⁹⁰Y, ¹⁸⁸Re, and ¹⁸⁶Re, and to a lesser extent ¹⁹⁹Au and ⁶⁷Cu. ¹³¹I has also been used. With the exception of ¹³¹I, all the methods currently used to conjugate these radiometals to antibodies involve the use of chelating groups chemically attached to the antibody. ⁶⁷Cu is one radionuclide that has been specifically recommended for use as a therapeutic radionuclide when bound to antibodies (DeNardo GL, Raventos A, Hines HH, *et al*. Requirements for a treatment planning system for radioimmunotherapy. *Int J Radiol Oncology Biol Phys* 11:335-348, 1985). ¹⁹⁹Au-conjugated monoclonal antibodies have also been suggested for potential use as cancer therapeutic agents.

⁶⁷Cu has been attached to monoclonal antibodies through chelates, e.g., a macrocycle chelate (6-para-nitrobenzyl-1,4,8,11-tetraazacyclotetradecane-N,N',N'',N''') (Deshpande SV, DeNardo SJ, Meares CF, *et al*. Copper-67-labeled monoclonal antibody Lym-1, A potential radiopharmaceutical for cancer therapy: labeling and biodistribution in RAJI tumored mice. *J Nucl Med 29*:217-225, 1988), and porphyrins (Roberts JC, Figard SD, Mercer-Smith JA, *et al*. Preparation and characterization of copper-67 porphyrin-antibody conjugates. *J Immunol Meth* 105:153-164, 1987). The macrocycle chelate, but not the porphyrin conjugate, was evaluated in an animal model system. Both ⁶⁴Cu and ⁶⁷Cu, have been conjugated by the porphyrin method to antibodies and autoantigenic peptides (Roberts JC, Newmyer SL, Mercer-Smith JA, Schrerer and Lavallee DK. Labelling antibodies with copper radionuclides using N-4-nitrobenzyl-5-(4-carboxyphenyl)-10,15,20-tris(4-sulfophenyl) porphine. *Appl Radiat* *Isot* 40:775-781, 1989). Biodistribution studies of radiocopper-labeled antibodies have shown that blood clearance is rapid and uptake to the bone is low (Mercer-Smith JA, Cole DA, Roberts JC, *et al*. The biodistribution of radiocopper-labeled compounds. In: C Kies (ed), *Copper Bioavailability and Metabolism*, pp 103-121, 1990).

Antibodies have been labeled with ¹⁹⁹Au, in the form of gold clusters (Hainseld JF, Foley CJ, Srivastava SC, *et al*. Radioactive gold cluster immunoconjugates: Potential agents for cancer therapy. *Nucl Med Biol* 17:287-294, 1990), and with ¹⁹⁹Au and ¹⁹⁵Au, as complex ions in citrate buffered saline (Anderson P, Vaugan ATM, and Varley NR. Antibodies labeled with ¹⁹⁹Au: Potential use of ¹⁹⁹Au for radioimmunotherapy. *Nucl Med Biol 15*:293-297, 1988).

Antibodies and other proteins have been directly labeled. Although several direct methods have been reported, the first direct method capable of providing a sufficiently strong bond between the protein and technetium-99m for *in vivo* applications was the direct or pretinning method described in U.S. Pat. No. 4,424,200, entitled *Method for Radiolabeling Proteins with Technetium-99m*, to Crockford, D.R., and Rhodes, B.A. In this method, a single reduction compound, consisting of stannous [Sn(II)] chloride and other salts which serves both to reduce the protein, thereby exposing the disulfide bonds, and to reduce the sodium pertechnetate, is used. With this method, many proteins can be successfully radiolabeled with ^{99m}Tc. Several investigators have reported on the use of this method (Rhodes, B.A., et al, "Technetium-99m labeling of murine monoclonal antibody fragments," *J Nucl Med* 27:685-693, 1986; Som, P., et al, "Radioimmunoimaging of experimental thrombi in dogs using technetium-99m-labeled monoclonal antibody fragments reactive with human platelets," *J Nucl Med* 27:1315-1320, 1987).

Equivalent methods for direct labeling have been reported (Schwarz, A., and Steinstruaber, A., "A novel approach to Tc-99m-labeled monoclonal antibodies," *J Nucl Med* 28:721, 1987; Pak, K.Y., et al, "A rapid and efficient method for labeling IgG antibodies with Tc-99m and comparison to Tc-99m Fab'". *J Nucl Med* 30:793, 1989; Granowska, M., et al, "A Tc-99m-labeled monoclonal antibody, PR1A3, for radioimmunoscintigraphy," *J Nucl Med* 30:748, 1989). In the equivalent methods disulfide reducing agents other than stannous salts were used. Pak et al used dithiothreitol to reduce the disulfide bonds of the antibody; Swartz and Steinsbruaber, and Granowska et al used 2-mercaptoethanol. Also some of these investigators (Swartz and Steinsbruaber, and Granowska et al) reduced the Tc-99m prior to adding it to the reduced antibody, which adds steps to the original procedure.

Reno, J.W., et al, U.S. Pat. No. 4,877,868, *Radionuclide Antibody Coupling*, uses dithiothreitol (DTT) to reduce the disulfide groups of the protein, then protect the reactive sulfides with Zn (II) or other sulfhydryl group derivatizing reagents. Tartrate salts are used to complex and transfer the reduced radionuclide. This method uses potentially toxic chemicals, such as dithiothreitol, to reduce the antibody. It also requires multiple steps to radiolabel the protein.

Thakur, M.L., U.S. Pat. No. 5,011,676, *Method to Directly Radiolabel Antibodies for Diagnostic Imaging and Therapy*, used sodium ascorbate to reduce the disulfide groups of antibodies. However, this method cannot be adapted to single-step, direct labeling; it is required to reduce the radionuclide prior to adding the radionuclide to the sodium ascorbate reduced protein. In a preferred embodiment of the Thakur method, a separate vial is utilized, in which sodium dithionite is used to reduce the radionuclide, producing dithionite reduced radionuclide.

There are useful metals for magnetic resonance imaging, including gadolinium, maganese, copper, iron, gold and europium, which are not radioisotopes. Examples also include ions of a lanthinide element of atomic numbers 57-70 or ions of transition metals of atomic numbers 21-29 and 42-44. Examples of metals which would be expected to be of potential utility in magnetic resonance imaging with proteins labeled by the methods described in the present invention include copper, iron and gold, as well as colloidal preparations of iron or gold.

So far, antibodies do not appear to have been labeled with positron-emitting radiometals, although other types of proteins (transferrin and human serum albumin) have been labeled with ⁶⁸Ga (Green MA, and Welch MJ. Gallium radiopharmaceutical chemistry. *Nucl Med Biol* 16:435-448, 1989). The short half-life associated with ⁶⁸Ga i.e., 68 minutes, suggests that it often may not be a suitable label for targeting antibodies, which tend to have prolonged biological half-lives.

### Peptides as Radiopharmaceuticals

The use of biologically active peptides, which are peptides which bind to specific cell surface receptors, has received some consideration as radiopharmaceuticals. Canadian Patent Application 2,016,235, *Labeled Chemotactic Peptides to Image Focal Sites of Infection or Inflammation*, teaches a method of detecting a site of infection or inflammation, and a method for treating such infection or inflammation, by administration of a labeled or therapeutically-conjugated chemotactic peptide. In this application, the chemotactic peptides are chemically conjugated to DTPA and subsequently labeled with ¹¹¹In. The utility of DTPA chelates covalently coupled to polypeptides and similar substances is well known in the art. Hnatowich, DJ, U.S. Pat. Nos. 4,479,930 and 4,668,503. Other bifunctional chelates for radiolabeling peptides, polypeptides and proteins are well known in the art. Other biologically active peptides described include that disclosed by Olexa SA, Knight LC and Budzynski AZ, U.S. Pat. No. 4,427,646, *Use of Radiolabeled Peptide Derived From Crosslinked Fibrin to Locate Thrombi In Vivo*, in which iodination is discussed as a means of radiolabeling. In Morgan CA Jr and Anderson DC, U.S. Pat. No. 4,986,979, *Imaging Tissue Sites of Inflammation*, use of chelates and direct iodination is disclosed. In Tolman GL, U.S. Pat. No. 4,732,864, *Trace-Labeled Conjugates of Metallothionein and Target-Seeking Biologically Active Molecules*, the use of metallothionein or metallothionein fragments conjugated to a biologically active molecule, including peptides, is disclosed. The previous methods all employ some conjugation means with a bifunctional chelator in order to effectuate labeling with a radionuclide or other medically useful metal ion, such as a paramagnetic contrast agent. The only exception involves radioiodination; the iodine labeling of proteins or peptides containing tyrosine or histidine residues is well known, for example, by the chloramine-T, iodine monochloride, Iodogen or lactoperoxidase methods.

Other biologically active peptides include analogs of formyl peptide chemoattractants which bind to neutrophils. These peptides are based on the sequence N-formyl-Met-Leu-Phe. The "C" terminal end can be modified to include additional sequences constituting a metal ion binding domain. The clinical and diagnostic imaging potential of formylated chemotactic peptides has recently been demonstrated by Fischman et al. (Fischman AJ, Pike MC, Kroon D, Fucello AJ, Rexinger D, tenKate C, Wilkinson R, Rubin RH and Strauss HW: Imaging focal sites of bacterial infection in rats with indium-111-labeled chemotactic peptide analogs. *J Nucl Med* 32:483-491, 1991) using chemotactic peptides chemically conjugated to DTPA and subsequently labeled with ¹¹¹In. Chemotactic peptides have also been radioiodinated by synthesizing formylated peptides containing tyrosine amino acids. These peptides have been used *in vitro* and have the same biological function as unlabeled formylated peptides (Janeczek AH, Marasco WA, Van Alten PJ and Walter RB: Autoradiographic analysis of formylpeptide chemoattractant binding, uptake and intracellular processing by neutrophils. *J Cell Sci* 94:155-168, 1989).

Peptide analogues of somatostatin have been used after radiolabeling for diagnostic imaging. Somatostatin is a hormone produced by the hypothalamus which normally inhibits the release of pituitary growth hormone. A number of peptide analogues have been developed which have pharmacological actions that mimic the naturally-occurring hormone. Octreotide acetate, one of the somatostatin analogues, has a disulfide bond in it. In normal subjects somatostatin and its analogues have the ability to suppress secretion of serotonin and the gastroenteropancreatic peptides, and growth hormone. A number of tumor types have been found to express somatostatin receptors, with ¹²⁵I-labeled somatostatin analogues used to image small-cell lung cancer (Kwekkeboom DJ, Krenning EP, Bakker WH et al: Radioiodinated somatostatin analog scintigraphy in small-cell lung cancer. *J Nucl Med* 32:1845-1848, 1991).

The potential role of amino acid sequences found in peptides and proteins in binding transition metals has been recognized. In Vallee BL and Auld DS: Zinc coordination, function, and structure of zinc enzymes and other proteins, *Biochemistry* 29:5648-5659, 1990, the general characteristics of non-metallothionein proteins which contain zinc binding sites are described. Arnold FH and Haymore BL describe histidine-containing amino acid sequences used for protein purification by metal-chelate chromatography (Engineered metal-binding proteins: purification to protein folding, *Science* 252:1796-1797, 1991). Iverson et al. describe a means of genetic manipulation of antibodies to contain metal binding sites in the immunological binding region with the goal of producing catalytic antibodies (Iverson BL, Iverson SA, Roberts VA, Getzoff ED, Tainer JA, Benkovic SJ and Lerner RA: Metalloantibodies, *Science* 249:659-662, 1990). The use of histidine-containing amino acid sequences which bind Ru to form exchange-inert metal complexes to form highly stable α-helical metallopeptides was described in Ghardiri MR and Fernholz AK: Peptide architecture. Design of stable α-helical metallopeptides via a novel exchange-inert Ru^{III} complex, *J Am Chem Soc* 112:9633-9635, 1990. The role of isolated amino acid ligands to bind ⁹⁹Tc and ^{99m}Tc has long been recognized; Seifert et al. describes the capability of nitrogen donor atoms to stabilize reduced technetium species using free lysine, ornithine and histidine (Seifert S, Munze R and Johannsen B: Technetium-99 and 99m chelates with N-donor ligands: a new class of potentially cationic radiopharmaceuticals, in *Technetium in Chemistry and Nuclear Medicine* Deutsch E, Nicolini M and Wagner HN Jr, eds., Cortina International, Verona, 1983, pp 19-23.

### Lung Imaging

Pulmonary radionuclide imaging techniques currently in general practice involve the use of a) macroaggregated albumin or albumin microspheres (MAA), b) radioaerosols (^{99m}Tc-DTPA), c) radioactive gases, and d) gallium citrate. See, generally, Anger, K: Radionuclide Studies of the Lung, **In**: Sperber, M. (editor), Radiologic Diagnosis of Chest Disease, Springer-Verlag, New York, 1990, pp 140-153; Miller RF and O'Doherty MJ: Pulmonary nuclear medicine. *Eur J Nucl Med* 19(1992) 355-368. MAA is a radioactive particle which is sequestered by capillary blockade. After administration of radioactive particles greater than 10 µm in diameter into a peripheral vein, the pulmonary capillaries and precapillary arterioles act like a sieve, with the ^{99m}Tc-MAA particles temporarily blocked so that the tracer is trapped in its first passage through the lung. The radioactivity distribution reveals relative pulmonary perfusion. When blood flow has been interrupted or significantly changed in a portion of the lung larger than 2 cm, a defect appears as a photon-deficient image. Most of the MAA particles (90%) have a diameter ranging from 10-40 µm. MAA particles degrade into smaller particles leaving the lung vasculature with a biologic half-time of 2-9 hours, and are cleared by phagocytosis in the reticuloendothelial system.

Many pulmonary diseases produce an altered pulmonary blood flow in the affected areas. Radiolabeled MAA allows detection of areas of altered blood flow, but provides no information related to any specific biochemical or metabolic event. Perfusion lung scintigraphy is highly sensitive, but not specific. Moreover, perfusion defects (13%) have been found in non-smoking volunteers without pulmonary disease, and loss of normal apex-to-base gradients (9%) have also been observed (Webber MM, Renick LH, Fouad BI, and Victery WK : Variants of the normal lung scan: Correlation with pulmonary function tests. *J Nucl Med* 13 (1972) 476; Tetalman MR, Hoffer PB, Heck LL, et al: Perfusion scans in normal volunteers. *Radiology* 106(1973) 593-594). The lack of specificity can lead to mis-diagnosis, the need for additional test procedures, and delays in implementing therapy. Thus, there is a need for a diagnostic radiopharmaceutical which can overcome some or all of these problems.

The lung is an organ which can undergo extensive degradation and remodeling of the extracellular matrix as a result of disease. Emphysema, fibrosis, cancer and other chronic obstructive lung diseases all can lead to both microscopic and macroscopic alterations in air space, and related changes in the lung extracellular matrix and basement membrane. A radiopharmaceutical which can detect specific alterations in extracellular molecules or their receptors can be used as a specific probe of the biochemical and metabolic status of the lung in disease processes.

Laminin is a basement membrane glycoprotein (Mᵣ = 900,000) which has various biological activities including promoting cell attachment, growth, and differentiation. A typical laminin molecule consists of three polypeptide chains -- A (440 kd), B1 (200 kd), and B2 (220 kd) -- that are linked by disulfide bonds to form an asymmetric cross-structure. Multiple, distinct adhesive sequences in laminin appear to mediate specific biological functions, and bind to distinct cell surface receptors (Hynes RO: Integrins: versatility, modulation, and signaling in cell adhesion, *Cell* 69(1992) 11-25; Yamada KM: Adhesive recognition sequences, *J Biol Chem* 266(1992) 2809-2812).

One adhesive sequence from the laminin A-chain is Ile-Lys-Val-Ala-Val (IKVAV), and this peptide as well as longer laminin peptide sequences containing IKVAV have been reported to increase in vitro adhesiveness of a number of cell lines including mast cells (Thompson HL, Burbelo PD, Yamada Y, Kleinman HK, and Metcalfe DD: Identification of an amino acid sequence in the laminin A chain mediating mast cell attachment and spreading, *Immunology* 72(1991) 144-149; Thompson HL, Burbelo PD, Yamada Y, Kleinman HK, and Metcalfe DD: Mast cells chemotax to laminin with enhancement after IgE-mediate activation, *J Immunol* 143(1989) 4188-4192), cerebral cells (Tashiro K-I, Sephel GC, Weeks B, Sasaki M, Martin GR, Kleinman HK, and Yamada Y: A synthetic peptide containing the IKVAV sequence from the A chain of laminin mediates cell attachment, migration, and neurite outgrowth, *J Biol Chem* 27(1989) 16174-16182; Kleinman HK, Weeks BS, Cannon FB, Sweeney TM, Sephel GC, Clement B, Zain M, Olson MOJ, Jucker M, Burrous BA: Identification of a 100-kDa nonintegrin cell surface laminin-binding protein which recognizes an A chain neurite-promoting peptide. *Arch Biochem Biophys* 290(1991) 320-325; Skubitz APN, Letourneau PC, Wayner E, and Furcht LT: Synthetic peptides from the carboxyl-terminal globular domain of the A chain of laminin: their ability to promote cell adhesion and neurite outgrowth, and interact with heparin and the B1 integrin subunit, *J Cell Biol* 115(1991) 1137-1148; Sephel GC, Tashiro K-I, Sasaki M, Greatorex D, Martin GR, Yamada Y, and Kleinman HK: Laminin A chain synthetic peptide which supports neurite outgrowth, *Biochem Biophys Res Comm* 162(1989) 821-829), normal mesenchymal cells (Kleinman et al., *supra*, 1991), tumor cells (Kleinman et al., *supra*, 1991), and hepatocytes (Clement B, Segui-Real B, Savagner P. Kleinman HK, and Yamada Y: Hepatocyte attachment to laminin is mediated through multiple receptors, *J Cell Biol* 110(1990) 185-192). One such longer peptide, Cys-Ser-Arg-Ala-Arg-Lys-Gln-Ala-Ala-Ser-Ile-Lys-Val-Ala-Val-Ser-Ala-Asp-Arg (also referred to as PA22-2), increased in vivo lung colonization by melanoma cells (Kanemoto T, Reich R, Royce L, Greatorex D, Adler SH, Shiraishi N, Martin GR, Yamada Y, and Kleinman HK: Identification of an amino acid sequence from the laminin A chain that stimulates metastasis and collagenase IV production, *Proc Nat Acad Sci (USA)* 87(1990) 2279-2283). The conformational status, but not specific chirality, of the IKVAV domain is a contributing factor in biological activity (Nomizu M, Utani A, Shiraishi N, Kibbey MC, Yamada Y, and Roller PR: The all-D-configuration segment containing the IKVAV sequence of laminin A chain has similar activities to the all-L-peptide *in vitro* and *in vivo*, *J Biol Chem* 267(1992) 14118-14121).

### Thrombus Imaging

Under homeostatic conditions, platelets circulate as disc shaped cells that do not interact with other circulating blood cells or vascular endothelium (Buchanan MR: Mechanisms of pathogenesis of arterial thrombosis: potential sites of inhibition by therapeutic compounds, *Sem Thrombosis and Hemostasis* 14(1988) 33-40). The release of adhesive and coagulant agents associated with platelet activation is held in check by high intraplatelet, and possibly vascular endothelium, levels of cAMP.

Upon injury, platelets rapidly attach a) to dysfunctional or detached endothelial cells and b) to the underlying basement membrane and tissues. Differences in platelet response, correlating to the degree of injury, are due in part to differences in the vessel wall composition of the molecules to which the platelets adhere. For example, type I and III collagens, which are typically associated with smooth muscle cells, promote platelet adhesion, aggregation, and release. In contrast, types IV and V collagens, typically associated with the endothelium, facilitate platelet adhesion but do not generally cause platelet activation.

Platelet-mediated thrombosis is a major pathogenetic mechanism in thrombogenesis and reocclusion after successful thrombolytic therapy, and consequently platelets are frequently used as vehicles for localization of thrombi. Additionally, suppression of platelet aggregation is a frequent target for prevention of blood vessel occlusion or reocclusion. There are a number of clinical conditions in which there are platelet accumulations; these include venous thrombosis, arterial thrombosis, left ventricular thrombosis, pulmonary embolism, inflammatory response secondary to myocardial infarction, endocarditis, bypass graft occlusion, aneurysms, prosthetic arterial graft platelet accumulation or occlusion, cerebral embolism or hemorrhage, traumatic injury with hemorrhage, gastrointestinal hemorrhage, and thrombosis secondary to catheters and other implanted devices.

A variety of diagnostic modalities have been used for conditions involving platelet accumulation. These include contrast venography, impedence plethysmography, and ¹²⁵I-fibrinogen uptake for venous thromboembolism; ¹¹¹In-labeled platelets for a variety of conditions involving platelet accumulation; and, pulmonary angiography, perfusion lung scanning using ^{99m}Tc-human macroaggregated albumin, and ventilation-perfusion lung scanning with radioactive gases or aerosols for pulmonary embolism. Each of these modalities presents serious limitations, and has less than desirable efficacy. ¹¹¹In-labeled platelets is the only modality which yields a reliable direct measure of platelet accumulation; however, this method suffers serious limitations, including technical difficulties in ex vivo labeling. In addition, since with ¹¹¹In-labeled platelets the labeling is performed ex vivo, and the platelets reinjected and allowed to accumulate before imaging, this method does not provide a measure of existing platelet accumulation. Thus, no commonly used method allows for direct detection of existing platelet accumulation within the body.

Peptides containing the adhesive sequence RGD are under active investigation as anti-thrombotic agents (Imura Y, Stassen J-M, Dunting S, Stockmans F, and Collen D: Antithrombotic properties of L-cysteine, N-(mercaptoacetyl)-D-Tyr-Arg-Gly-Asp-sulfoxide (G4120) in hamster platelet-rich femoral vein thrombosis model, *Blood* 80(1992) 1247-1253). Knight et al. (Knight LC, Radcliffe R, Kollman M, Dasika V, Wikander R, Mauer AH, Rodwell JD, and Alvarez V: Thrombus imaging with Tc-99m synthetic peptides reactive with activated platelets. *J Nucl Med* 31(1990) 757 (abstract)) have reported on the use of ^{99m}Tc-synthetic peptide-metallothionein complexes which bind to the platelet glycoprotein IIb/IIIa complex to image fresh thrombi in jugular veins. However, peptides which target the glycoprotein IIb/IIIa complex are known to adversely affect platelet aggregation, and consequently a radiopharmaceutical based on such an approach would be expected to have severe dose limitations.

In addition to peptides, radiolabeled monoclonal antibodies specific for platelet-related antigens have been studied as diagnostic radiopharmaceuticals. (Shah VO, Zamora PO, Mills SL, Mann PL, and Comp PC: In vitro studies with the platelet-reactive antibody 50H.19 and its fragments. *Thrombosis Research* 58(1990) 493-504; Som P, Oster ZH, Yamamoto K, Sacker DF, Brill AB, Zamora PO, Newell KD, and Rhodes BA: Radioimmunoimaging of experimental thrombi in dogs using Tc-99m labeled monoclonal antibody fragments reactive with human platelets. *J Nucl Med* 27(1986) 1315-1320).

Integrin-type receptors on platelets (glycoprotein Ib, the glycoprotein IIb/IIIa complex and glycoprotein IV) have been identified as the major adhesion receptors in platelets, but these glycoproteins do not appear to play a role in the interaction of platelets with the intact laminin molecule (Tandon NN, Holland EA, Kralisz U, Kleinman HK, Robey FA, and Jamieson GA: Interaction of human platelets with laminin and identification of the 67 kDa laminin receptor on platelets, *Biochem J* 274(1991) 535-542). However, platelets do bind to laminin peptide fragments via these receptors (Sonnenberg A, Gehlsen KR, Aumailley M, and Timpl R: Isolation of a6b1 integrins from platelets and adherent cells by affinity chromatography on mouse laminin fragment E8 and human laminin pepsin fragment, *Exp. Cell Res.* 197(1991) 234-244), suggesting that normally these sites in laminin are cryptic for platelets. One non-integrin platelet receptor for laminin is a 67 kDa receptor which binds to laminin-derived peptide sequences containing Tyr-Ile-Gly-Ser-Arg (YIGSR) (Tandon et al., *supra*). This platelet receptor appears to play an important role in the interaction of platelets with the intact laminin molecule. Platelet adherence to laminin via this receptor does not in itself result in platelet activation (Ill CR, Engvall E, and Ruoslahti E: Adhesion of platelets to laminin in the absence of activation. *J Cell Biol*. 99(1984) 2140-2145).

Peptides containing the YIGSR peptide sequence have been proposed as anti-metastatic agents. Yamada Y, Graf JO, Iwamoto Y, Rober F, Kleinman HK, Sasaki M and Martin GR, U.S. Patent 5,092,885, *Peptides with Laminin Activity*; Schasteen CS, U.S. Patent 5,039,662, *Peptide with Anti-Metastatic Activity*. These patents involve longer sequences containing the YIGSR peptide sequence, as well as acylated YIGSR peptide sequences.

### Summary of the state of the art

The labelling method of US 4,424,200 (*supra.*) which is confined to proteins, describes the labelling of e.g. IgG by incubating the protein with buffered stannous chloride and then further incubating an aliquot of the resultant solution with radioactive sodium pertechnetate. There is no clear explanation of the mechanism involved. However it is implicit that a single reducing agent consisting of stannous (Sn(II)) chloride serves both to reduce the protein, thereby exposing the disulfide bonds, and to reduce the pertechnetate labelling agent. The process described involves no separation of excess reducing agent and no proposals are made involving transition metals other than tin.

Labelling of peptides with metal ions has heretofore involved the use of bifunctional chelators, e.g. WO 90/13317. A number of publications given above have described certain potentially useful sequences for therapy but without any proposals as regards labelling.

### The invention

The labelling of peptides, particularly small peptides which can be easily synthesised, is subject to much greater difficulty than is the case with large molecules which have numerous sites available. The number of sites for labelling may as small as one or two per molecule (as compared to hundreds or thousands) and much greater certainty is required. The stability problem is magnified enormously. Only a method which is simple in concept and follows a clearly understood mechanism can hope to achieve reproducibility. At the same time, the method must result in stable products and possess the potential for wide application to be successful in a commercial sense. The invention follows the discovery of such a method, following extensive research on the mechanism involved and also includes claims to radiolabelled specific peptides having a structure dictated by the method of the invention.

According to one aspect of the invention there is provided a method of preparing a pharmaceutical composition containing a labelled peptide suitable for administration to a patient, characterised by:
(a) obtaining or preparing a peptide substrate comprising a biological function domain (BD) and a medically useful labelling metal ion binding domain (MD), said substrate having a formula selected from:
   (R₁) - [Y₁]ₙ - (R₂),
   (R₁) - [Y₁ - (R₂) - Y₁]ₙ - (R₃) and
   (R₁) - [Y₁ - (R₂) - Y₂]ₙ - (R₃)
   where
   R₁, R₂ and R₃ each comprise an amino acid sequence containing from 0 to 20 amino acids,
   Y₁ and Y₂ are amino acids containing S, N and/or O capable of complexing with ions of a transition metal selected from Zn, Cu, Sn, Co and Ni, and
   n is an integer from 1 to 6
      wherein
      said MD is selected from [Y₁]ₙ, [Y₁ - (R₂) - Y₁]ₙ and [Y₁ - (R₂) - Y₂]ₙ and
      said BD comprises at least one of R₁, R₂ and R₃ and includes an amino acid sequence containing from 1 to 20 amino acids.
(b) reacting said substrate in a pharmaceutically acceptable aqueous buffer solution with a source of ions of a said transition metal so as to form complexes of said S, N and/or O-containing amino acid and said transition metal ions, and
(c) reacting said complexed substrate in said buffer solution with a medically useful labelling metal ion, said labelling ion having a higher order of binding than said transition metal ion so as to replace the transition metal ion by said labelling metal ion on said MD, thereby producing said composition comprising a labelled peptide in said buffer,
   provided that where said substrate includes disulfide bonds these are first reduced by incubation of the substrate with a reducing agent and excess reducing agent is afterwards removed.

The metal ion binding domain (MD) preferably includes one or more amino acid sequences containing monosulfide groups without disulfide bonds, e.g. selected from cysteine, histidine, penicillamine, deacylated methionine, lysine, arginine, aspartic acid, glutamic acid and tyrosine.

Preferably the MD is selected from
[Cys - (R₂) - Cys]ₙ
[Cys - (R₂) - Pen]ₙ
[His - (R₂) - Cys]ₙ
[His - (R₂) - Pen]ₙ
[His]ₙ and
[His - (R₂) - His]ₙ
where n is from 1 to 6 and R₂ is an amino acid sequence containing from 1 to 20 amino acids.

The biological function domain (BD) may be selected from:
Phe - Trp - Lys - Thr,
N-formyl - Met - Leu - Phe,
Tyr - Ile - Gly - Ser - Arg (YIGSR) and
Ile - Lys - Val - Ala - Val (IKVAV)

Preferred peptide sequences include:
Arg - Lys - Gln - Ala - Ala - Ser- Ile - Lys - Val - Ala - Val,
   (RKQAASIKVAV)
Cys-Ser-Arg-Ala-Arg-Lys-Gln-Ala-Ala-Ser-Ile-Lys-Val-Ala-Val-Ser-Ala-Asp-Arg,
   (CSRARKQAASIKVAVSADR)
Cys - Asp - Pro - Gly - Tyr - Ile - Gly - Ser - Arg
   (CDPGYIGSR)

The preferred transition metal ion is stannous (Sn(II). The source of the stannous ions may comprise a member selected from stannous tartrate, stannous glucoheptonate, stannous gluconate, stannous phosphonate, stannous chloride and stannous fluoride.

The preferred labelling metal ion is Tc. In this case, or in general where the transition metal ion is stannous, a reducing agent may be added during step (c) to reduce the oxidation state of labelling metal ion which may be pertechnetate.

The said buffer solution may comprise anions of one or more dicarboxylic acids preferably selected from phthalate, tartrate and citrate. Where the transition metal ion is stannous the said buffer solution preferably includes alkali metal tartrate at a pH from 5 to 6. The preferred buffer solution consists essentially of a mixture of 10 mM tartrate and 40 mM phthalate. It may also contains an amino acid such as glycine.

The said labelling ion is preferably selected from ions of Fe, Co, Ni, Cu, Zn, As, Se, Mo, Tc, Ru, Pd, Ag, Cd, In, Re, Os, Ir, Pt, Au, Hg, Tl, Pb, Bi, Po and At, especially the radioactive ions of Tc, Re, Cu, Ag and In.

Preferably the composition prior to step (c) is stored in dosage vials in lyophilised form and rehydrated during or immediately prior to step (c).

According to a second aspect the invention provides a pharmaceutical composition containing a labelled peptide as defined above suitable for administration to a patient, said peptide comprising a biological function domain (BD) and a medically useful labelling metal ion binding domain (MD) comprising at least one amino acid containing sulfur, nitrogen and/or oxygen capable of complexing with ions of a transition metal selected from Zn, Cu, Sn, Co and Ni, and a medically useful labelling metal ion, characterised in that:
(a) said MD is as defined in any one of claims 1 to 4, and
(b) said BD comprises one of:
   Tyr - Ile - Gly - Ser - Arg (YIGSR) and
   Ile - Lys - Val - Ala - Val (IKVAV)
(c) said labelling metal ion is directly coupled to said sulfur, nitrogen and/or oxygen of said MD.

The peptide preferably contains one or more sequences selected from
Arg - Lys - Gln - Ala - Ala - Ser- Ile - Lys - Val - Ala - Val,
   (RKQAASIKVAV)
Cys-Ser-Arg-Ala-Arg-Lys-Gln-Ala-Ala-Ser-Ile-Lys-Val-Ala-Val-Ser-Ala-Asp-Arg,
   (CSRARKQAASIKVAVSADR) and
Cys - Asp - Pro - Gly - Tyr - Ile - Gly - Ser - Arg
   (CDPGYIGSR)

The labelling metal ion is preferably as defined above and the peptide is preferably present in an aqueous buffer as already defined.

According to another aspect, the invention provides a lyophilised or frozen dosage unit containing a targeting agent suitable for administration to a patient and ready (upon rehydration) for direct labelling by the addition of radioactive pertechnetate or perhenate,
characterised in that said agent comprises a peptide as defined above comprising a biological function domain (BD) and a medically useful labelling metal ion binding domain (MD), wherein
(a) said MD is as above defined, and
(b) said BD comprises one of:
   Tyr - Ile - Gly - Ser - Arg (YIGSR) and
   Ile - Lys - Val - Ala - Val (IKVAV)

The peptide preferably contains one or more sequences selected from
Arg - Lys - Gln - Ala - Ala - Ser- Ile - Lys - Val - Ala - Val,
   (RKQAASIKVAV)
Cys-Ser-Arg-Ala-Arg-Lys-Gln-Ala-Ala-Ser-Ile-Lys-Val-Ala-Val-Ser-Ala-Asp-Arg,
   (CSRARKQAASIKVAVSADR) and
Cys - Asp - Pro - Gly - Tyr - Ile - Gly - Ser - Arg
   (CDPGYIGSR)

The peptide is preferably present in an aqueous buffer as defined above.

According to another aspect the invention provides a composition in dosage form containing a targeting agent suitable for administration to a patient consisting of rehydrated contents of a dosage unit as defined above which have been labelled by the addition of a radioactive labelling metal ion.

The labelling metal ion is preferably radioactive pertechnetate.

The invention is inclusive of the use of a peptide as defined above for the preparation of a composition as defined above, or of a dosage unit as defined above for administration to a patient for diagnostic imaging of the lung, wherein said BD comprises the sequence
Ile - Lys - Val - Ala - Val (IKVAV).

The peptide preferably contains the sequence
Arg - Lys - Gln - Ala - Ala - Ser- Ile - Lys - Val - Ala - Val,
   (RKQAASIKVAV) or
Cys-Ser-Arg-Ala-Arg-Lys-Gln-Ala-Ala-Ser-Ile-Lys-Val-Ala-Val-Ser-Ala-Asp-Arg,
   (CSRARKQAASIKVAVSADR)

More preferably the peptide consists essentially of the sequence
Cys-Ser-Arg-Ala-Arg-Lys-Gln-Ala-Ala-Ser-Ile-Lys-Val-Ala-Val-Ser-Ala-Asp-Arg.
   (CSRARKQAASIKVAVSADR)
including repetitions of IKVAV.

The invention is also inclusive of the use of a peptide as defined above for the preparation of a composition as defined above, or of a dosage unit as defined above, for administration to a patient for the detection of sites of platelet accumulation or carcinomas, wherein said BD comprises the sequence
Tyr - Ile - Gly - Ser - Arg (YIGSR)

Preferably the peptide also contains the sequence
Asp - Pro - Gly

The preferred peptide consists essentially of the sequence
Cys - Asp - Pro - Gly - Tyr - Ile - Gly - Ser - Arg
   (CDPGYIGSR)
including repetitions of YIGSR.

The invention is also inclusive of the use of a peptide as defined above containing thiolate groups complexed through the sulfur atoms of the thiolate groups with ions of a transition metal selected from Zn, Cu, Sn, Co and Ni, for direct labelling by reaction of the complexed peptide in a pharmaceutically acceptable buffer with a medically useful labelling metal ion having a higher order of binding than said transition metal ion, whereby said labelling metal ions replace said complexed transition metal ions resulting in a composition comprising a labelled peptide in said buffer. Preferably said transition metal ions are stannous ions.

Preferred forms of the invention are described hereinafter.

Using the methods of this invention, peptides containing metal ion binding sequences can be coupled directly with metal ions to provide materials useful for in vivo diagnostic and therapeutic applications. Peptides may be used alone, in combination with other peptides or may be chemically-conjugated to a host molecule. The peptides can be prepared in a format providing a labelling kit which can, in turn, be used to prepare a metal ion-peptide complex for in vivo use. The peptides of this invention contain:
a) biological-function domains, and
b) metal ion-binding domains which can complex with medically useful metal ions.

The biological-function domain of the peptide is a sequence of one or more amino acids which exhibit binding to a biological receptor found on cells, tissues, organs or fluids. The peptides may or may not transmit a signal to the cells, tissues or other materials associated with the biological receptor after binding. The biological-function domain also includes a sequence of one or more amino acids which exhibit binding to a biological receptor found on other peptides, enzymes, antibodies or similar proteinaceous compositions which may themselves exhibit binding to another biological receptor.

The biological-function domain may be located in any one or more of R₁, R₂ or R₃, including situations in which the biological-function domain comprises all or part of two or more of R₁, R₂ or R₃. It is not required that the biological-function domain constitute all of the amino acid sequence of any one of R₁, R₂ or R₃; that is, it is possible and contemplated that the biological-function domain will be an amino acid sequence constituting a portion of the total amino acid sequence of any one of R₁, R₂ or R₃, with the remainder of that region being an amino acid sequence which is not the biological-function domain.

The metal ion-binding domain of the peptide is a sequence of one or more amino acids containing sulfur, nitrogen or oxygen which is capable of complexing with metal ions. Sulfur-containing amino acids include primarily cysteine (Cys), cystine (Cys-Cys) and penicillamine (Pen), although deacylated methionine (Met) may also be used. Nitrogen-containing amino acids include primarily histidine (His), but under certain conditions lysine (Lys) and arginine (Arg), which have p*K*ₐ values of 10.0 and 12.0, may also be employed. In addition, the terminal amino group of peptides may also be employed. Oxygen-containing amino acids include aspartic acid (Asp), glutamic acid (Glu) and tyrosine (Tyr), as well as the terminal carboxyl group of peptides. The amino acid sequences most usefully employed will include one or more Cys, one or more His, or a combination of Cys and His. Pen, which is an analogue of Cys, may be directly substituted for any given Cys. Cys may be present in the peptide as a disulfide in the form of cystine. The metal ion-binding domains may occur once or multiple times in any given peptide, and may occur in any combination. The metal ion-binding domain and the biological-function domain may overlap.

The metal binding sequences as found in the peptides of this invention are stabilized by the addition of a positively-charged transition metal ion of Zn, Cu, Sn, Co, or Ni, selected to have a low order of binding strength. Through a replacement reaction, the transition metal ion replaces the H ion of the thiolate, imidazole or carboxyl group. The divalent ions of zinc and tin are thought to be particularly attractive. The transition metals are weakly associated with the peptide.

There are two primary peptide configurations which require somewhat different methods in order to achieve stable labelling with a metal ion. One peptide configuration involves a metal ion-binding domain which includes one or more disulfide bonds. The most common example of this is 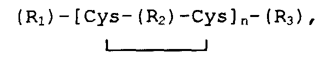
(R₁)-[Cys-(R₂)-Cys]ₙ-(R₃),
 wherein [Cys-(R₂)-Cys]ₙ is the medically useful metal ion-binding domain, which can appear in the amino acid sequence from 1 time to about 6 times; and R₁, R₂ and R₃ are each amino acid sequences containing from 0 to about 20 amino acids, with at least one of the amino acid sequences R₁, R₂ and R₃ containing the biological-function domain. An example of a peptide fragment meeting this criteria is 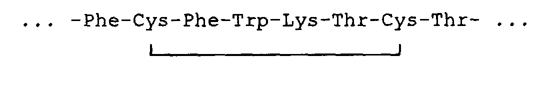
... -Phe-Cys-Phe-Trp-Lys-Thr-Cys-Thr- ...
 in which the biological-function domain is R₂, being the sequence Phe-Trp-Lys-Thr; the metal ion-binding domain is [Cys-(R₂)-Cys]ₙ, wherein n equals 1, being the sequence Cys-Phe-Trp-Lys-Thr-Cys; R₁ is the sequence ... -Phe; and R₃ is the sequence Thr- ... Other peptide configurations in which reducible disulfide bonds are present are also included in this method. These include the substitution of Pen for one or both Cys amino acids, as well as the modification of a native Met to allow it to form a disulfide bond. The biological-function domain can appear in any one of R₁, R₂ and R₃, and can also span more than one region, so that the biological-function domain may constitute, for example, R₂ and R₃, or some portion of R₂ and R₃. Any one or more of the regions R₁, R₂ and R₃ may contain no amino acids. Examples of peptides which contain disulfide bonds include e.g. somatostatin and its analogues.

In those peptides in which the metal ion-binding domain includes one or more disulfide bonds, it is necessary to first reduce the disulfide bond or bonds.

Numerous reducing agents have been described and are known to those skilled in the art. Particularly useful types of reducing agents include 2-mercaptoethanol; 1,4-dithiothreitol; 2,3-dihydroxybutane-1,4-dithiol; 2-aminoethanethiol HCl; 2-mercaptoethylamine; thioglycolate; cyanide; cysteine; reduced glutathione; Sn(II); Cu(I); and Ti(II). The reducing agent may be dissolved in a solute or may be attached to a solid phase. Reducing agents attached to a solid phase are commercially available, and methods for their use are known to those skilled in the art. The degree to which the peptide requires disulfide bond reduction depends on the nature of the peptide and its intended medical application. In any event, reduction is halted before excessive fragmentation of the peptide or loss of the biological-function of the peptide occurs.

In one specific embodiment, Sn(II) is used as a reducing agent at a concentration of 5 mM. In this embodiment the Sn(II) is dissolved in a buffer composed of approximately 10 mM tartrate and 40 mM phthalate, pH 5.5, and the Sn(II) buffer admixed with a peptide substrate at a concentration of 8.3 mg/ml. The reduction reaction is allowed to proceed for a period of time at room temperature, three hours having been employed successfully with some peptides containing a single disulfide bond, after which time the reaction is terminated by removing excess Sn(II) ions by molecular sieve chromatography. One means of molecular sieve chromatography employs Sephadex G-25, with the chromatography gel pre-equilibrated, and the peptide eluted in 0.9% NaCl or other suitable buffer.

Removal of the reducing agent, whether Sn(II) or some other reducing agent, can be accomplished by a variety of suitable means, including such methods as dialysis, ultrafiltration, positive-pressure membrane filtration, precipitation, preparative high performance liquid chromatography, affinity chromatography, other forms of chromatography and preparative isoelectric focusing. Many of the reducing agents contain thiols, which if present in the final labelling mixture, can complex with the medically useful metal ion. Such complexes can have severe and unknown side effects if administered in vivo. Additionally, some reducing agents exhibit unacceptable toxicity. Thus removal of the reducing agent both limits the degree of reduction to that desired, as well as providing for increased utility and safety of the labelled preparation by removal of toxic or otherwise undesirable reducing agents.

Thiolate groups in reduced peptides are highly reactive and can interact to reform disulfide bonds. The use of Sn(II) is believed to minimise the reformation of disulfide bonds. Sources of Sn(II) include stannous tartrate, stannous glucoheptonate, stannous gluconate, stannous phosphonate, stannous chloride, and stannous fluoride. The selection of the source of Sn(II) and its final concentration depends on the intended medical application of the peptide, the nature of the peptide, the relative and absolute number of thiolate groups and the metal ion to be used. In one embodiment stannous tartrate is used at a concentration of 1.25 mM. The stannous tartrate is added to the peptide after removal of the peptide-reducing agent. The stannous tartrate is prepared in a buffer composed of 10 mM tartrate and 40 mM phthalate, pH 5.6, and is added to peptide to yield a final concentration of 1 mg/ml peptide solution.

Sn(II) can be stabilized by use of dicarboxylic acids, such as phthalate and tartrate. A wide range of dicarboxylic acids, known to those skilled in the art, may be similarly used to stabilise the Sn (II) and/or to act as a buffer. If the phthalate and tartrate are in molar excess relative to the Sn(II), then these dicarboxylic acids also stabilise the medically useful metal ion in a form which can react with the peptide. In one embodiment tartrate and phthalate are used in the Sn(II) agent at concentrations of 10 mM and 40 mM, respectively.

Similarly, the Sn(II) and the medically useful metal ion may be stabilized by free amino acids used singly or in combination with other agents. The type of amino acid used and the specific concentration depends on the nature of the peptide and its intended use. In one embodiment, glycine is used at a concentration of 0.1-10 mM, and in another, histidine is used at a concentration of 0.1-10 mM.

The peptide may be stored frozen in bulk form after disulfide bond reduction and the removal of excess reducing agent. Alternatively, (as described below in connection with the other method not involving reduction of disulfide bonds) the peptide may be stored in bulk form or in unit dose form after addition of the Sn(II).

The other peptide configuration involves one or more amino acids containing sulfur, nitrogen or oxygen which is available for binding, or which can be made available for binding to metal ions. Commonly used amino acids include Cys, Pen and His, or any combination of them. This peptide configuration does not involve initial reduction of disulfide bonds (sulfur being present as monosulfide). The simplest case takes the form
(R₁)-[Cys]ₙ(R₂)
wherein [Cys]ₙ is the medically useful metal ion-binding domain and ₙ is typically a number between 1 and about 6; and R₁ and R₂ are each an amino acid sequence containing from 0 to about 20 amino acids, with at least R₁ and R₂ including the biological-function domain. In this and all related forms, it should be noted that R₁ and R₂ are interchangeable; either can contain the biological-function domain, the biological-function domain may include part or all of both R₁ and R₂, and the biological-function domain may constitute only a portion of the amino acid sequence in either R₁ or R₂. The order of components for these purposes can be varied, so that (R₁)-[Cys]ₙ-(R₂), (R₂)-[Cys]ₙ-(R₁), [Cys]ₙ-(R₂)-(R₁), [Cys]ₙ-(R₁)-(R₂) and the mirror images of the last two orderings are all equivalent, even though the resulting peptides may significantly differ in other aspects. A representative example of this form is the sequence
Cys-Asp-Pro-Gly-Try-Ile-Gly-Ser-Arg
in which the Cys is [Cys]ₙ wherein ₙ is 1, Tyr-Ile-Gly-Ser-Arg is the biological-function domain (R₁) and Asp-Pro-Gly is (R₂), so that the structure of the sequence is [Cys]ₙ-(R₂)-(R₁).

Other forms of the same general configuration include
(R₁)-[Cys-(R₂)-Cys]ₙ-(R₃),
(R₁)-[Cys-(R₂)-Pen]ₙ-(R₃),
(R₁)-[His-(R₂)-Cys]ₙ-(R₃),
(R₁)-[His-(R₂)-Pen]ₙ-(R₃),
and (R₁)-[His-(R₂)-His]ₙ-(R₃)
wherein the sequence [...]ₙ is the medically useful metal ion-binding domain with n typically being a number between 1 and about 6; and R₁, R₂ and R₃ are each an amino acid sequence containing from 0 to about 20 amino acids, with at least one of R₁, R₂ and R₃ including the biological-function domain. Here too the ordering is irrelevant to the functional description; for example, mirror images of the foregoing two orderings, all orderings in which the positions of His and Cys are reversed, and orderings in which the biological-function domain is present in the any of the three regions R₁, R₂ and R₃, any portion of the three regions R₁, R₂ and R₃, or any combination of the three regions R₁, R₂ and R₃, are all equivalent to the third configuration listed above, (R₁)-[His-(R₂)-Cys]ₙ-(R₃). Each of the other foregoing configurations can be similarly described.

The positively-charged transition metal ions are introduced to the peptide in an aqueous solution containing an appropriate buffer. The buffer may consist of dicarboxylic acids (tartrate, phthalate, citrate), amino acids (glycine), borate or the like. For radiolabelling in acidic conditions typically 10 mM tartrate and 40 mM phthalate, pH 5.6, are used. For radiolabelling in basic conditions typically 10 mM glycine, pH 9.0, is used. The buffer may also contain a number of excipients and/or stabilisers including NaCl, inositol, glucoheptonate, or the like.

The peptides are subsequently incubated with a medically-useful metal ion. The medically-useful metal ion is selected to have a higher order of binding than the positively charged-transition metal ion used to stabilise the metal binding sequences. A number of medically-useful metal ions can be used; radiometals include isotopes of the elements of Tc, Re, Au, Ag, Pd, As, Cu, Hg, and Ru. Radioisotopes of Tc are of significant interest, and particularly ^{99m}Tc. In the case of ^{99m}Tc, the peptides are reacted with sodium pertechnetate which has been treated with a reducing agent to generate Tc with a lower oxidation state. The product of the reaction between the metal ion and the peptide is a complex of the metal ion and the peptide. For example, the following structures could result from use of the invention, using Tc labelling of peptides containing metal-ion binding domains consisting of Cys and His groups as an example: 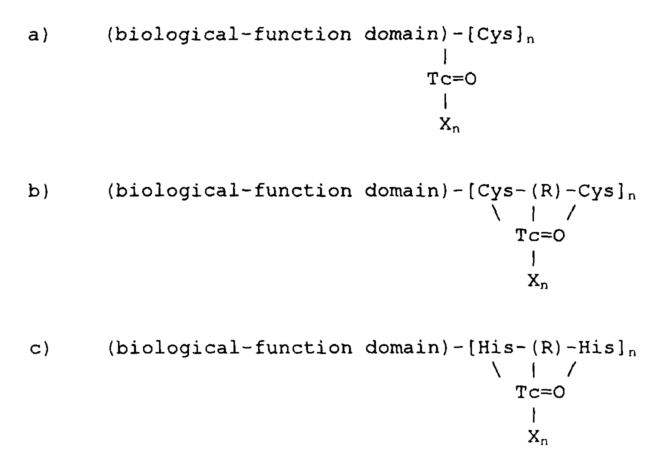 in which R is an amino acid sequence containing from 0 to about 20 amino acids and Xₙ is an anion, such as a halogen like fluoride or chloride, or a solvent molecule, such as water.

The resulting Tc-peptide bond should have a sufficiently high bond strength to minimise the exchange of the radionuclide to transferrin and serum albumin. The complex should be thermodynamically stable under physiological conditions and exhibit acceptable toxicological properties.

Most stannous reductions are performed at a pH of from about 5 to about 6. With amino acid side chains in a solution at pH 5.6, the basic amino acids are positively charged, the acidic amino acids are largely negatively charged, the alcoholic amino acids are neutral, and methionine is neutral. Since reduced technetium binds more readily to neutral hydrogen donors rather than positively charged hydrogen donors, at the pH range 5 to 6 only Cys and His are optimal ^{99m}Tc binding site candidates. For both Cys and His, radiolabelling yields are dependant on pH, and are theoretically optimal at or near the p*K*ₐ.

The preferred transition metal is Sn(II); useful sources of Sn(II) include stannous tartrate, stannous glucoheptonate, stannous gluconate, stannous phosphonate, stannous chloride, and stannous fluoride. The selection of the source of Sn(II) and its final concentration depends on the intended medical application of the peptide, the nature of the peptide, the relative and absolute number of thiolate groups and the metal ion to be used. In one embodiment stannous tartrate is used at a concentration of 1.25 mM. The stannous tartrate is prepared in a buffer composed of 10 mM tartrate and 40 mM phthalate, pH 5.6, and is added to peptide to yield a final concentration of 1 mg/ml peptide solution.

As is the case in the method involving reduction of disulfide bonds, Sn (II) can be stabilized by use of dicarboxylic acids, such as phthalate and tartrate. A wide range of dicarboxylic acids, known to those skilled in the art, may be similarly used to stabilise the Sn(II) and/or to act as a buffer. If the phthalate and tartrate are in molar excess relative to the Sn(II), then these dicarboxylic acids also stabilise the medically useful metal ion in a form which can react with the peptide. In one embodiment tartrate and phthalate are used in the Sn(II) agent at concentrations of 10 mM and 40 mM, respectively.

Similarly, the Sn(II) and the medically useful metal ion may be stabilized by free amino acids used singly or in combination with other agents. The type of amino acid used and the specific concentration depends on the nature of the peptide and its intended use. In one embodiment, glycine is used at a concentration of 0.1-10 mM, and in another, histidine is used at a concentration of 0.1-10 mM.

The peptide may be stored in bulk form or in unit dose form after addition of the Sn(II) or other transition metal. For example, in one embodiment the peptide is stored at -20°C in vials after introduction of the Sn(II). Methods used in lyophilisation of peptides are known to those skilled in the art. Either frozen or lyophilised preparations may be maintained for an indefinite period before labelling by the addition of the medically useful metal ion.

In both the frozen and lyophilised storage forms, excipients may be added to the peptide to minimise damage which can arise from ice-crystal formation or free-radical formation. The type of excipient and the concentration depends on the nature of the peptide and the intended use. In one embodiment, glycine and inositol are used as excipients in lyophilised preparations.

A typical lyophilised preparation made by the embodiments set forth above would, upon rehydration, contain 10 mM tartrate, 40 mM phthalate, 22 µg of Sn(II), 500 µg of peptide, 2 mg/ml of glycine, and 2 mg/ml of inositol. To label with a medically useful metal ion, a typical lyophilised preparation is hydrated by the addition of a solution containing 0.9% NaCl (U.S.P.) or water for injection (U.S.P.) and the medically useful metal ion.

Alternatively, it is possible to hydrate the lyophilised preparation, and to add the metal ion in a subsequent step. If a frozen preparation is used, it is thawed and allowed to come to room temperature, and a solution containing the medically useful metal ion is then added. The nature and amount of the medically useful metal ion and the specific reaction conditions depend on the isotopic nature of the metal, and the intended medical application. In one embodiment, ^{99m}Tc is added in the form of pertechnetate ion in a solution of 0.9% NaCl. The ^{99m}Tc is typically incubated for up to 30 minutes to insure completion of the reaction with the peptide, after which the radiolabelled preparation can be directly used in medical applications. In another embodiment, ⁶⁷Cu is added in a solution of 10 mM tartrate and 40 mM phthalate at pH 5.6. In yet another embodiment, ¹⁸⁸Re or ¹⁸⁶Re is added to a solution of 10 mM tartrate and 40 mM phthalate, at pH 5.6, and containing Sn(II), and then heated to lower the oxidation state of Re. The resulting solution is then added to the lyophilised or frozen preparation.

In the embodiment in which ^{99m}Tc is used, the Sn(II) is present in the peptide-containing solution in sufficient excess to alter the oxidation state of the Tc ion such that it can bind to ionisable groups. Similar approaches may be used to lower the oxidation state of other medically useful metal ions for subsequent binding to ionisable groups. The type of the metal ion, its isotopic nature, and concentration would depend on the intended medical application.

The metal ion-peptides of this invention may be used directly for administration, or alternatively may be conjugated to a carrier or targeting molecule. The methods for conjugating peptides to carrier molecules are well known to those skilled in the art. The conjugations may involve covalent binding through carbohydrate residues, sulfhydryl residues, amine groups (including those of lysine), and carboxyl groups.

The peptides of the invention can be:
a) naturally-occurring,
b) produced by chemical synthesis,
c) produced by recombinant DNA technology,
d) produced by biochemical or enzymatic fragmentation of larger molecules,
e) produced by methods resulting from a combination of a-d, or
f) produced by any other means for producing peptides.

The peptide of this invention is reacted with a medically useful metal ion. The medically useful metal ion may be radioactive and generate gamma rays, beta particles, or positrons which are converted into gamma rays upon collision with electrons. Alternatively, the medically useful metal ion may be paramagnetic. The medically useful metal ion may used in diagnostic imaging procedures including gamma scintigraphy, specific photon emission computerised tomography, or positron emission tomography. The medically useful metal ion may also be used diagnostically in magnetic resonance imaging. Medically useful metal ions may also be used therapeutically.

The type of medically useful metal ion depends on the specific medical application. Particularly useful metal ions can be found in the group consisting of elements 26-30 (Fe, Co, Ni, Cu, Zn), 33-34 (As, Se), 42-50 (Mo, Tc, Ru, Rh, Pd, Ag, Cd, In, Sn) and 75-85 (Re, Os, Ir, Pt, Au, Hg, Tl, Pb, Bi, Po, At). Isotopes of the elements Tc, Re, and Cu are particularly applicable for use in diagnostic imaging and radiotherapy. The isotope ^{99m}Tc is particularly applicable for use in diagnostic imaging. Other radionucleides with diagnostic or therapeutic applications include ⁶²Cu, ⁶⁴Cu, ⁶⁷Cu, ⁹⁷Ru, ¹⁰⁵Rh, ¹⁰⁹Pd, ¹⁸⁶Re, ¹⁸⁸Re, ¹⁹⁸Au, ¹⁹⁹Au, ²⁰³Pb, ²¹¹Pb and ²¹²Bi.

The product resulting from the methods set forth herein can be used for both medical applications and veterinary applications. Typically, the product is used in humans, but may also be used in other mammals. The primary applications of the invention involve human patients, but the invention may be applied to laboratory, farm, zoo, wildlife, pet or sport animals.

The product may be used to monitor normal or abnormal metabolic events, to localise normal or abnormal tissues, to localise diseases, and to bind to blood constituents, including blood cells, such as lymphocytes, for subsequent localisation of diseases, infections, and abnormal tissues. The application and medical use of the product depends on the type of peptide and the type of medically useful metal ion used.

Using the methods of this invention, peptides with a biological-function domain comprising at least the sequence IKVAV and a linked radiolabel provide materials useful for in vivo diagnostic applications, particularly for diagnostic imaging of the lungs. Preferably, the peptide comprises a biological-function domain of at least the sequence IKVAV and a metal-ion binding domain comprising metal ion binding sequences which can be coupled directly with metal ions. The peptides can be prepared in a format providing a labelling kit which can, in turn, be used to prepare a metal ion-peptide complex for in vivo use. It is also possible to provide for labelling of a peptide with the biological-function domain with a metal ion in vivo, such as through use of a peptide-avidin complex, which is injected in vivo, followed by a biotin-metal ion complex inject in vivo, resulting in formation of an in vivo peptide-avidin-biotin-metal ion complex.

The IKVAV-containing peptides of this invention preferably contain:
a) biological-function domains comprising at least the sequence IKVAV, and
b) metal ion-binding domains which can complex with medically useful metal ions.

The biological-function domain of the IKVAV-containing peptide is a sequence of the amino acids Ile-Lys-Val-Ala-Val (IKVAV single amino acid code), and optionally amino acids in addition to IKVAV which are useful for lung imaging and treatment. The IKVAV peptide of this invention will, for the most part, include the sequence RKQAASIKVAV, and most preferably the sequence CSPARKQAASIKVAVSADR. Usually, within the indicated sequences, there may be mutations, including deletions, insertions or substitutions. It is possible that the sequence IKVAV may be repeated one or more times, to increase localisation. For the most part, substitutions will be conservative, in which amino acids having substantially the same conformation and polarity may be employed. The peptides may use L-amino acids, or one or more of the amino acids may be substituted by D-amino acids (D-stereoisomer), which may in part increase resistance to protease degradation. Particularly, one or more alanines may be substituted. In the alternative, terminal amino acids may be employed having unnatural chirality. The peptide may also include a terminal amide or a terminal acylated amino acid, particularly acetylated or alkylated, particularly methylated, amino acids. Where a cysteine provides the metal-ion binding domain at the N-terminus, the cysteine may be alkylated or unsubstituted on the mercaptan group.

It is hypothesised, that lung localisation using the IKVAV-containing peptide is receptor-based, and due in part to pulmonary endothelial cell binding, and in some instances to tumor receptor binding. There is also evidence to suggest that IKVAV-containing peptide binds to receptors on tissue plasminogen activator, which is frequently present in relative high concentrations in tumor cells. Regardless of the exact mechanism of receptor-based lung localisation, such a mechanism presents significant advantages for a ^{99m}Tc-peptide containing the IKVAV sequence over ^{99m}Tc-MAA:
a) The peptide should not itself alter pulmonary perfusion,
b) The peptide should bind to pre-capillary, capillary, and post-capillary endothelial cells and thereby, provide a more representative view of the actual physiology of the lung vasculature,
c) The use of a synthetic peptide for imaging would obviate considerations relating to viral (HIV or the like) contamination of the source material,
d) The use of a non-particulate imaging agent should minimise health risks in hyper-sensitive patient populations, such as pediatric use;
e) Differential diagnosis of certain conditions may be possible, in that chronic obstructive conditions such as emphysema will be detected as photon-deficient, and certain tumors will be detected as photon-rich.

The IKVAV-containing peptide product may be used to monitor or treat normal or abnormal tissues and metabolic events, particular chronic obstructive pulmonary disease, such as emphysema or fibrosis, in which abnormal tissues or metabolic events will generally produce a photon-deficient area, and to localise primary or metastatic cancerous tumors, and particularly cancerous tumors of the lung, in which cancerous tumors will generally produce a photon-abundant area.

Using the methods of this invention, peptides with a biological-function domain comprising at least the sequence YIGSR and a linked radiolabel provide materials useful for in vivo diagnostic applications, particularly for diagnostic imaging of thrombosis and other conditions characterised by accumulation of platelets. Preferably, the peptide comprises a biological-function domain comprising at least the sequence YIGSR and a metal-ion binding domain comprising metal ion binding sequences which can be coupled directly with metal ions. The peptides can be prepared in a format providing a labelling kit which can, in turn, be used to prepare a metal ion-peptide complex for in vivo use. It is also possible to provide for labelling of a peptide with the biological-function domain with a metal ion in vivo, such as through use of a peptide-avidin complex, which is injected in vivo, followed by a biotin-metal ion complex inject in vivo, resulting in formation of an in vivo peptide-avidin-biotin-metal ion complex.

The YIGSR containing peptides of this invention preferably contain:
a) biological-function domains comprising at least the sequence YIGSR, and
b) metal ion-binding domains which can complex with medically useful metal ions.

The biological-function domain of the YIGSR-containing peptide is a sequence of the amino acids Tyr-Ile-Gly-Ser-Arg (YIGSR single amino acid code), and optionally amino acids in addition to YIGSR. The peptide of this invention thus preferably includes the sequence YIGSR, which may be repeated one or more times. Usually, within the indicated sequences, there may be mutations, including deletions, insertions or substitutions. For the most part, substitutions will be conservative, in which amino acids having substantially the same conformation and polarity may be employed. The peptides may use L-amino acids, or one or more of the amino acids may be substituted by D-amino acids (D-stereoisomer). Particularly, one or more alanines may be substituted. In the alternative, terminal amino acids may be employed having unnatural chirality. The peptide may also include a terminal amide or a terminal acylated amino acid, particularly acetylated or alkylated, particularly methylated, amino acids. Where a cysteine provides the metal-ion binding domain at the N-terminus, the cysteine may be alkylated or unsubstituted on the mercaptan group.

The YIGSR-containing peptide product may be used to monitor or treat normal or abnormal tissues and metabolic events characterised by accumulation of cells with receptors for YIGSR-containing peptides, which accumulations will generally produce a photon-abundant area using most imaging modalities, particularly those involving detection of gamma rays. Most commonly, the product will be used to detect accumulations of platelets. There are a number of clinical conditions in which there are platelet accumulations; these include venous thrombosis, arterial thrombosis, left ventricular thrombosis, pulmonary embolism, inflammatory response secondary to myocardial infarction, endocarditis, bypass graft occlusion, aneurysms, prosthetic arterial graft platelet accumulation or occlusion, cerebral embolism or hemorrhage, traumatic injury with hemorrhage, gastrointestinal hemorrhage, and thrombosis secondary to catheters and other implanted devices.

The resulting product of this invention can be used in a variety of medical procedures including gamma scintigraphy, specific photon emission computerised tomography, positron emission tomography, and magnetic resonance imaging. It is also possible to use the product to deliver a therapeutic quantity of radiation to a disease site. The medical application of the product of this invention depends on the type of peptide and the type of medically useful metal ion used.

The invention is further illustrated by the following examples.

### EXAMPLE 1 - CHEMOTATIC PEPTIDE ANALOGUE

### N-formyl-Met-Leu-Phe-Gly-His-Gly-Gly-His-Gly-His-Gly-Gly-His

This peptide is a chemotactic peptide analogue, specifically an analogue of N-formyl-Met-Leu-Phe, one of several peptides which are chemotactic for cells of the lymphatic system. The sequence His-Gly-Gly-His-Gly-His-Gly-Gly-His is used to bind Tc. The peptide was dissolved directly in 10 mM tartrate/40 mM phthalate buffer, pH 5.6 (P/T buffer), to result in a solution containing 1.4 mg of peptide/ml. This solution was mixed (7:3) with P/T buffer containing 1.25 mM stannous tartrate. Aliquots of 0.5 ml was then dispensed into individual vials. Each kit contained 0.5 mg of peptide, 40 mM phthalate, 10 mM tartrate, and 22 µg of stannous tartrate. All solutions were purged with nitrogen prior to use and all preparations prepared under an anaerobic atmosphere. The peptides in the labelling kits were labelled with ^{99m}Tc by addition of 1-2 mCi of sodium pertechnetate (U.S.P.) and allowing the reaction to proceed for 30 minutes.

### EXAMPLE 2 - COMPARATIVE POTENTIAL BINDING STUDY

To compare the potential binding of ^{99m}Tc to histidine and cysteine, ^{99m}Tc binding in three peptides with known amino acid sequences was evaluated. One peptide, with the amino acid sequence H₂N-Cys-Asp-Pro-Gly-Tyr-Ile-Gly-Ser-Arg, contained a single cysteine residue and no histidines, and was prepared by dissolving directly in 10 mM tartrate/40 mM phthalate buffer, pH 5.6 (P/T buffer), resulting in a solution containing 1.4 mg of peptide/ml. This solution was mixed (7:3) with P/T buffer containing 1.25 mM stannous tartrate. Aliquots of 0.5 ml was then dispensed into individual vials. Each vial contained 0.5 mg of peptide, 40 mM phthalate, 10 mM tartrate, and 22 µg of stannous tartrate. All solutions were purged with nitrogen prior to use and all preparations prepared under an anaerobic atmosphere. The peptide in the vials was labelled with ^{99m}Tc by addition of 1-2 mCi of sodium pertechnetate (U.S.P.) and allowing the reaction to proceed for 30 minutes.

Another peptide, with the sequence (Acetyl)-Asp-Arg-Val-Ile-His-Pro-Phe-His-Leu-Val-Ile-His-Asp, contained histidine residues but no cysteines or cystine, and was prepared and radiolabelled as set forth in Example 1. The control, poly-tyrosine, contained neither histidine nor cysteine; it was prepared and radiolabelled as set forth in the preceding paragraph.

The histidine-containing peptide bound some but not all the added ^{99m}Tc. The cysteine-containing peptide bound essentially all of the added ^{99m}Tc. Poly-tyrosine, the negative control material, did not label. These results were confirmed by conventional thin-layer chromatography.

### EXAMPLE 3 - SYNTHESIS OF PEPTIDE CONTAINING BIOLOGICAL-FUNCTION DOMAIN AND METAL ION-BINDING DOMAIN

The chemotactic peptide analogue N-formyl-Met-Leu-Phe-Gly-His-Gly-Gly-His-Gly-His-Gly-Gly-His was synthesised using a commercially available automated synthesiser. The peptide was lyophilised and purified using reverse phase HPLC. The peptide was then labelled using the methods of Example 1.

### EXAMPLE 4 - PREPARATION OF IKVAV-CONTAINING PEPTIDE KITS FOR ^{99m}Tc LABELLING

Laminin-derived peptide of the sequence CSRARKQAASIKVAVSADR was obtained commercially (Bachem, Inc.) as lyophilised powder and used without additional purification. The N-terminal thiolate associated with the Cys residue was used as the metal ion-binding domain for subsequent labelling with reduced ^{99m}Tc.

Peptide labelling kits were prepared aseptically using nitrogen-purged solutions, and whenever feasible under an atmosphere of nitrogen. To prepare the peptide labelling kits, the peptide was dissolved to a final concentration of 1.4 mg/ml in chilled, nitrogen-purged 10 mM tartrate/40 mM phthalate buffer, pH 5.6 (P/T buffer) containing 2% maltose. The peptide and P/T buffer solution was then mixed (7:3) with P/T buffer containing 1.25 mM stannous tartrate. Aliquots (typically 0.5 ml containing 500 µg of peptide) were then sterile filtered through a 0.22 micron filter, and dispensed into individual vials. The head space of each vial was purged with nitrogen, the vials stoppered and crimped, and stored frozen at -70°C.

### EXAMPLE 5 - ^{99m}Tc LABELLING OF IKVAV-CONTAINING PEPTIDE KITS

To radiolabel, a vial of the preparation of Example 4 was removed from the freezer and allowed to come to room temperature. The labelling reaction was initiated by the addition of 0.5 - 2.0 mCi of ^{99m}Tc (sodium pertechnetate in saline). Radiochemical analysis was begun 30 minutes after the introduction of the pertechnetate.

### EXAMPLE 6 - RADIOCHEMICAL ANALYSIS BY CHROMATOGRAPHY OF IKVAV-CONTAINING PEPTIDE KITS

To determine the relative amount of ^{99m}Tc bound to a given peptide preparation of Example 4, aliquots of the ^{99m}Tc-labelled preparations made by the method of Example 5 were analysed by molecular sieve HPLC, reverse phase chromatography, and thin layer chromatography.

Molecular sieve HPLC was performed using a 7.5 x 300 mm TSK G3000SW column preceded with a TSK-SW 7.5 x 7.5 mm guard column (TosoHaas, Philadelphia, PA) at a flow rate of 1 ml/minute of a phosphate buffered saline solution (0.01 M phosphate, pH 7.0, containing 0.15 M NaCl), with a UV and radioisotope detector in series. The ^{99m}Tc-IKVAV-containing peptide preparation eluted at 12.8 minutes with a low chromatographic recovery (less than 10%). In control studies, pertechnetate eluted at 17.8 minutes with essentially quantitative chromatographic recovery.

For reverse-phase analysis, Sep-Pak C₁₈ mini-columns (Millipore Inc., Bedford, MA) were used as reverse-phase adsorbents to evaluate the binding of ^{99m}Tc to the peptides. The columns were rinsed with 10 ml of 100% ethanol followed by 10 ml of 0.001% HCl. Aliquots of 100 µl of the test sample were loaded onto the column and the unbound material eluted with 10 ml of 0.001% HCl. The column was then serially eluted with a graded series of 10 ml solutions of aqueous ethanol (10%, 20%, 30%, 40%, 50%, 60%, and 100%). The radioactivity in each eluant fraction (0.001% HCl through 100% ethanol) was determined by counting an aliquot (20 µl) of each fraction in a gamma scintillation counter. The columns themselves were also counted, after allowing an appropriate time for decay. All counts were corrected for decay and the amounts of radioactivity in each fraction expressed as a percentage of the total radioactivity assayed. The reverse-phase chromatography using C₁₈ mini-columns eluted with a graded series of ethanol confirmed ^{99m}Tc binding to the peptide (Table 1).

TLC was used to measure the amount of peptide-bound (and unbound) ^{99m}Tc and the amount of radiolabelled aggregate/colloid. Both measurements involved the use of ITLC-SG (Gelman Sciences, #61886) chromatography paper, cut into 1.5 ' 10 cm strips and activated by heating for 30 minutes at 110°C, as per the manufacturer's instructions. After heating, the strips were stored at room temperature until use.

Peptide-bound ^{99m}Tc in the radiolabelled preparations was measured using TLC in 85% aqueous methanol using ITLC-SG strips. The solvent separated the soluble, unbound ^{99m}Tc (which migrates with the solvent front) from ^{99m}Tc bound to the peptide (which remains at the origin). Percentage of unbound ^{99m}Tc was expressed as CPM in the origin half of the strip divided by the total CPM, with all measures corrected for background.

Thin layer chromatography of the ^{99m}Tc-IKVAV-containing peptide preparation of Example 4 in saline over heat-activated silica-gel coated cellulose (ITLC-SG paper) showed essentially all radioactivity associated with the peptide (R_{f} = 0). The preparations did not contain significant amounts of unbound ^{99m}Tc as pertechnetate or ^{99m}Tc-tartrate (R_{f} = 1.0).

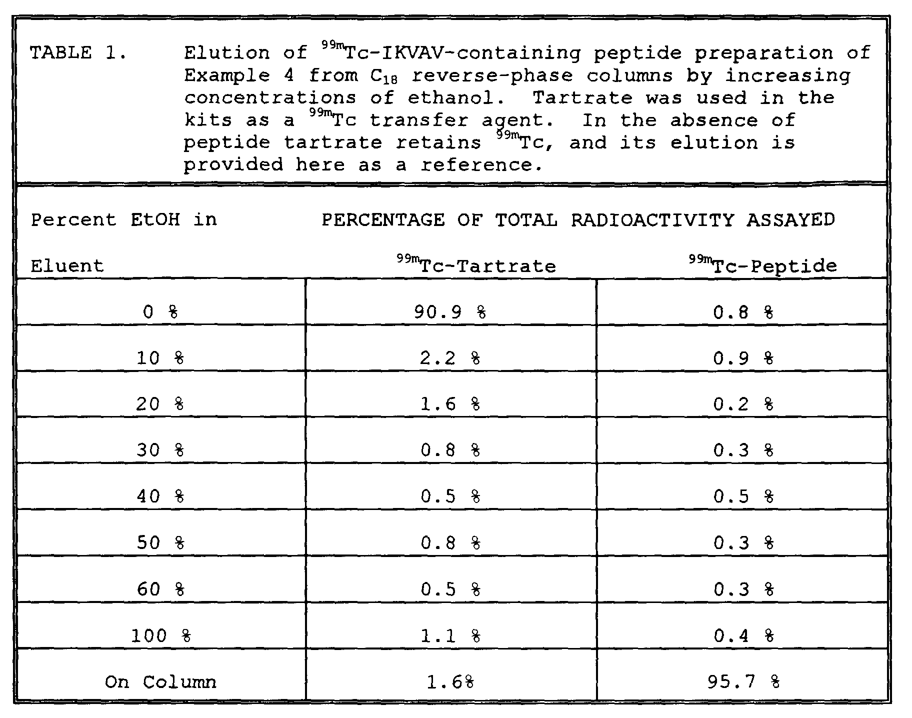

### EXAMPLE 7 - BIODISTRIBUTION IN RODENTS OF IKVAV-CONTAINING ^{99m}Tc-PEPTIDE

The biodistribution of the ^{99m}Tc-peptide of Example 4 was evaluated in adult female Swiss-Webster mice (approximately 19 g) at selected times (10, 30, and 120 minutes) after injection into the tail vein. Each experimental group was composed of at least five animals, with each animal receiving 0.1 ml containing 5 µg of peptide (1 µCi/µg). Animals were sacrificed by Halothane overdose, and selected organs dissected, weighed, and associated radioactivity determined. Data were analysed using a computer program specifically designed for ^{99m}Tc-labelled preparations. The percent dose per organ for blood, bone, and muscle were calculated assuming 7, 8.2, and 40% of total body weight, respectively, for these tissues.

Following the injection of ^{99m}Tc-IKVAV-containing peptide of Example 4, a significant amount of radioactivity was found in the lungs at both 10 and 30 minutes post injection (Table 2). Major accumulations were also found in the liver and kidneys. By two hours post injection the amount of radiolabel in the lung had fallen to less than 5% (from 47% at 10 minutes post injection, with a concomitant increase in kidney activity noted). Only small uptakes of ^{99m}Tc were noted in other organs.

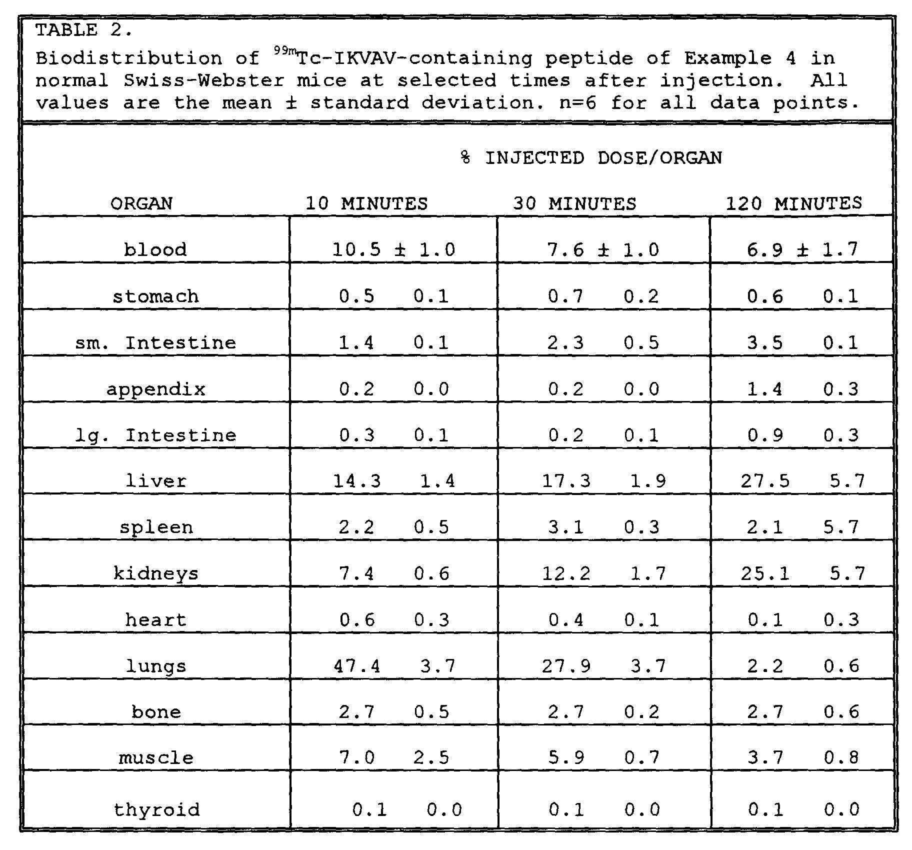

Some studies involved pre-incubation of the ^{99m}Tc-IKVAV-containing peptide in whole blood prior to injection and determination of biodistribution. In these studies, whole human blood was obtained from a healthy adult male donor and collected into Vacutainer tubes containing EDTA. After mixing to insure proper dissolution of the EDTA, approximately 2.5 ml of the whole blood was removed and mixed with 0.25 ml of ^{99m}Tc-peptide. The mixture was allowed to incubate 30 minutes at room temperature. After 30 minutes, aliquots of 0.1 ml were injected into the tail vein of the mice. The amount of radioactivity in the circulation for ^{99m}Tc-IKVAV-containing peptide pre-incubated in whole blood was higher than in animals receiving ^{99m}Tc-IKVAV-containing peptide without incubation in blood. With incubation of ^{99m}Tc-peptide in whole blood prior to injection, significantly decreased lung uptake was noted (Table 3).

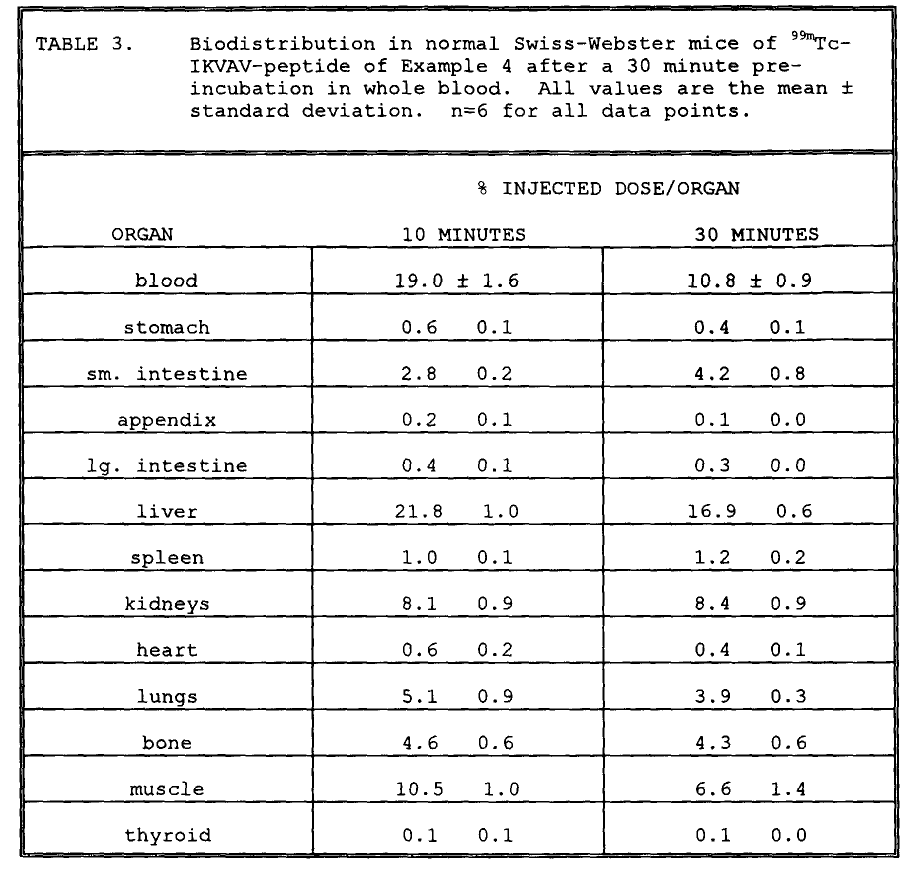

The clearance rates of the ^{99m}Tc-IKVAV-containing peptide of Example 4 was evaluated in adult female Fischer 344 rats at 2 hours after injection. Each experimental group was composed of three animals. Each animal was anaesthetised with ketamine and the bile duct and bladder were cannulated. Blood was collected over various periods of time from the jugular vein. The ^{99m}Tc-peptide cleared very rapidly from the plasma of rats, with a clearance rate of 2.4 ml/minute. At two hours, 10.8 ± 4.9 % of the injected dose had cleared through urine, while bile clearance at the same time point was 0.9 ± 0.3%.

### EXAMPLE 8 - DOSE RESPONSE OF ^{99m}Tc-IKVAV PEPTIDE ON LUNG LOCALISATION

The effect of the dose of ^{99m}Tc-IKVAV-containing peptide of Example 4 on lung localisation was evaluated. Localisation in the lung was found in all injection doses used (0.05, 0.5, and 5 µg) at both 10 and 30 minutes post injection. Similar amounts of radioactivity were found in the lungs regardless of the amount injected at 10 minutes post injection (110.6%, 111.9%, and 144.4% I.D./gram of lung tissue for 0.05, 0.5, and 5 µg, respectively). At 30 minutes post injection a more pronounced effect of dose was noted, with more radioactivity retained in the lung with a larger injection dose (48.8%, 68.1%, and 91.9% I.D./gram of lung tissue for 0.05, 0.5, and 5 µg, respectively).

### EXAMPLE 9 - EXAMINATION OF IKVAV-CONTAINING PEPTIDE KITS FOR PARTICULATE IMPACTION

Certain experiments were conducted to determine if high lung uptake resulted from the preparation of Example 4 forming a particle which impacts in the lung. No particles were visible when freshly radiolabelled ^{99m}Tc-IKVAV-peptide of Example 4 was examined under a phase contrast microscope, and ^{99m}Tc-IKVAV-peptide of Example 4 filtered through a submicron filter (0.2 micron pore size) still localised to the lungs. Additionally, when ^{99m}Tc-IKVAV-peptide of Example 4 was injected into the peritoneal cavity of mice (to sequester potential colloid), localisation to the lung was still found. In these experiments, the lung-heart ratio of ^{99m}Tc-peptide of Example 4 was elevated (3:1) at 15 minutes after i.p. injection, and increased so that by 60 minutes post injection the ratio was 9:1. At 120 minutes the ratio had decreased, but was still nearly 6:1.

### EXAMPLE 10 - BIODISTRIBUTION IN MELANOMA TUMOR BEARING MICE OF ^{99m}Tc-IKVAV-CONTAINING PEPTIDE

Biodistribution studies involving nude mice bearing melanoma tumors in the lung were conducted. Aliquots of B-16 melanoma cells were injected (50,000 cells in 0.1 ml serum-free RPMI medium) into the tail vein of adult nude mice and were used in studies approximately 3 weeks after inoculation. Paired studies were done using nude mice receiving sham injections of saline without cells.

The biodistribution of ^{99m}Tc-IKVAV-peptide of Example 4 was markedly altered in animals with tumors in the lung compared to those without tumors in the lung. In these studies, five tumored and five control animals were used for each time point, and results are ± the standard deviation. In tumored animals, the amount of lung uptake (% injected dose) was increased compared to controls at all time points examined, so that with tumored animals lung uptake was 38.3 ± 4.0 %, 28.1 ± 3.6 % and 3.9 ± 1.2 % at 10, 30, and 120 minutes post-injection, while with control nude mice at the same time points, the lung uptake was 19.0 ± 2.7 %, 11.9 ± 2.9 % and 1.6 ± 0.4 %, respectively.

### EXAMPLE 11 - BIODISTRIBUTION IN EMPHYSEMA MODEL MICE OF ^{99m}Tc-IKVAV-CONTAINING PEPTIDE

Biodistribution was also studied in a lung disease model which used tight-skin mice with genetic emphysema. The tight-skin (*Tsk*) mouse is a genetic mutant caused by a dominant gene deficiency of serum anti-elastase. Heterozygous (*Tsk/+*) animals show multiple skin connective tissue abnormalities resembling scleroderma as well as an increased growth of cartilage, bone, and small tendons with hyperplasia of the tendon sheaths. The *Tsk* trait is associated with progressive pulmonary emphysema and development of right ventricular hypertrophy, as well as with lung collagen changes. These mice, as well as genetic control mice (pallid), were obtained from The Jackson Laboratory (Bar Harbor, ME).

The relative localisation of ^{99m}Tc-IKVAV-peptide of Example 4 at all time points examined (10, 30, and 120 minutes post-injection), was decreased in the lungs of animals with emphysema relative to paired control animals.

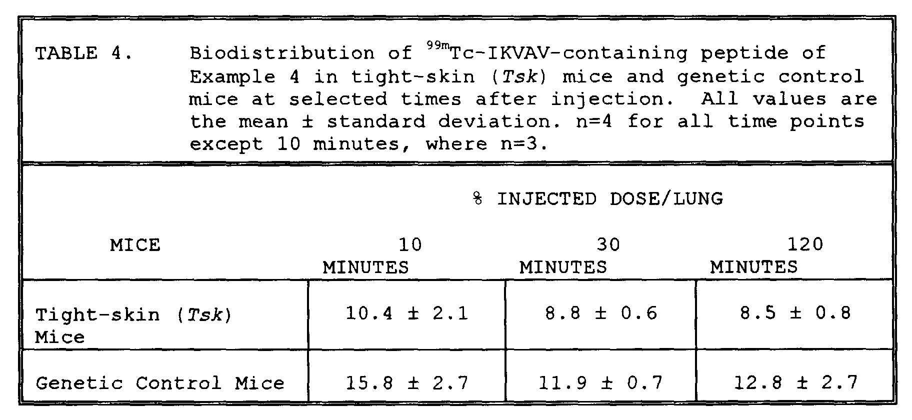

In a degenerative lung disease like emphysema the total number of receptors would be expected to decrease, due to loss of lung mass. In such a case, the amount of localisation of ^{99m}Tc-IKVAV peptide in the lungs would decrease relative to the localisation found in paired genetic control animals. The observations made correlate with this hypothesis.

### EXAMPLE 12 - PREPARATION OF YIGSR-CONTAINING PEPTIDE KITS FOR ^{99m}Tc LABELLING

Laminin-derived peptide of the sequence CDPGYIGSR (H₂N-Cys-Asp-Pro-Gly-Tyr-Ile-Gly-Ser-Arg) was obtained commercially (Bachem, Inc.) as lyophilised powder and used without additional purification. The N-terminal thiolate associated with the Cys residue was used as the metal ion-binding domain for subsequent labelling with reduced ^{99m}Tc.

Peptide labelling kits were prepared aseptically using nitrogen-purged solutions, and whenever feasible under an atmosphere of nitrogen. To prepare the peptide labelling kits, the peptide was dissolved to a final concentration of 1.4 mg/ml in chilled, nitrogen-purged 10 mM tartrate/40 mM phthalate buffer, pH 5.6 (P/T buffer) containing 2% maltose. The peptide and P/T buffer solution was then mixed (7:3) with P/T buffer containing 1.25 mM stannous tartrate. Aliquots (typically 0.5 ml containing 500 µg of peptide) were then sterile filtered through a 0.22 micron filter, and dispensed into individual vials. The head space of each vial was purged with nitrogen, the vials stoppered and crimped, and stored frozen at -70°C.

### EXAMPLE 13 - ^{99m}Tc LABELLING OF YIGSR-CONTAINING PEPTIDE KITS

To radiolabel, a vial of the preparation of Example 12 was removed from the freezer and allowed to come to room temperature. The labelling reaction was initiated by the addition of 0.5 - 2.0 mCi of ^{99m}Tc (sodium pertechnetate in saline). Radiochemical analysis was begun 30 minutes after the introduction of the pertechnetate.

### EXAMPLE 14 - RADIOCHEMICAL ANALYSIS BY CHROMATOGRAPHY OF ^{99m}Tc-LABELLED YIGSR CONTAINING PEPTIDE

To determine the relative amount of ^{99m}Tc bound to a given YIGSR-containing peptide preparation, aliquots of the ^{99m}Tc-labelled preparations of Example 13 were analysed by molecular sieve HPLC, reverse phase chromatography, and thin layer chromatography.

Molecular sieve HPLC was performed using a 7.5 x 300 mm TSK G3000SW column preceded with a TSK-SW 7.5 x 75 mm guard column (TosoHaas, Philadelphia, PA) at a flow rate of 1 ml/minute phosphate buffered saline (0.01 M phosphate, pH 7.0, containing 0.15 M NaCl), with a UV and radioisotope detector in series. The preparation of Example 12, labelled by the method of Example 13, eluted at 13.9 minutes with a high chromatographic recovery (greater than 95%). In control studies, pertechnetate eluted at 17.8 minutes with essentially quantitative chromatographic recovery.

For reverse-phase analysis, Sep-Pak C₁₈ mini-columns (Millipore Inc., Bedford, MA) were used as reverse-phase adsorbents to evaluate the binding of ^{99m}Tc to the peptides. The columns were rinsed with 10 ml of 100% ethanol followed by 10 ml of 0.001% HCl. Aliquots of 100 µl of the test sample were loaded onto the column and the unbound material eluted with 10 ml of 0.001% HCl. The column was then serially eluted with a graded series of 10 ml solutions aqueous ethanol (10%, 20%, 30%, 40%, 50%, 60%, and 100%). The radioactivity in each eluant fractions (0.001% HCl - 100% ethanol) was determined by counting an aliquot (20 µl) of each fraction in a gamma scintillation counter. The columns themselves were also counted, after allowing an appropriate time for decay. All counts were corrected for decay and the amounts of radioactivity in each fraction expressed as a percentage of the total radioactivity assayed.
Reverse-phase chromatography using C₁₈ mini-columns eluted with a graded series of ethanol confirmed ^{99m}Tc binding to the peptides (Table 5).

TLC was used to measure the amount of peptide-bound (and unbound) ^{99m}Tc and the amount of radiolabelled aggregate/colloid. Both measurements involved the use of ITLC-SG (Gelman Sciences, #61886) chromatography paper, cut into 1.5 ' 10 cm strips and activated by heating for 30 minutes at 110°C, as per the manufacturer's instructions. After heating, the strips were stored at room temperature until use.

Peptide-bound ^{99m}Tc in the radiolabelled preparations was measured using TLC in 85% aqueous methanol using ITLC-SG strips. The solvent separated the soluble, unbound ^{99m}Tc (which migrates with the solvent front) from ^{99m}Tc bound to the peptide (which remains at the origin). Percentage of unbound ^{99m}Tc was expressed as CPM in the origin half of the strip divided by the total CPM, with all measures corrected for background.

Thin layer chromatography of the preparation of Example 12 in saline over heat-activated silica-gel coated cellulose (ITLC-SG paper) showed essentially all radioactivity associated with the peptide (R_{f} = 0). The preparations did not contain significant amounts of unbound ^{99m}Tc as pertechnetate or ^{99m}Tc-tartrate (R_{f} = 1.0).

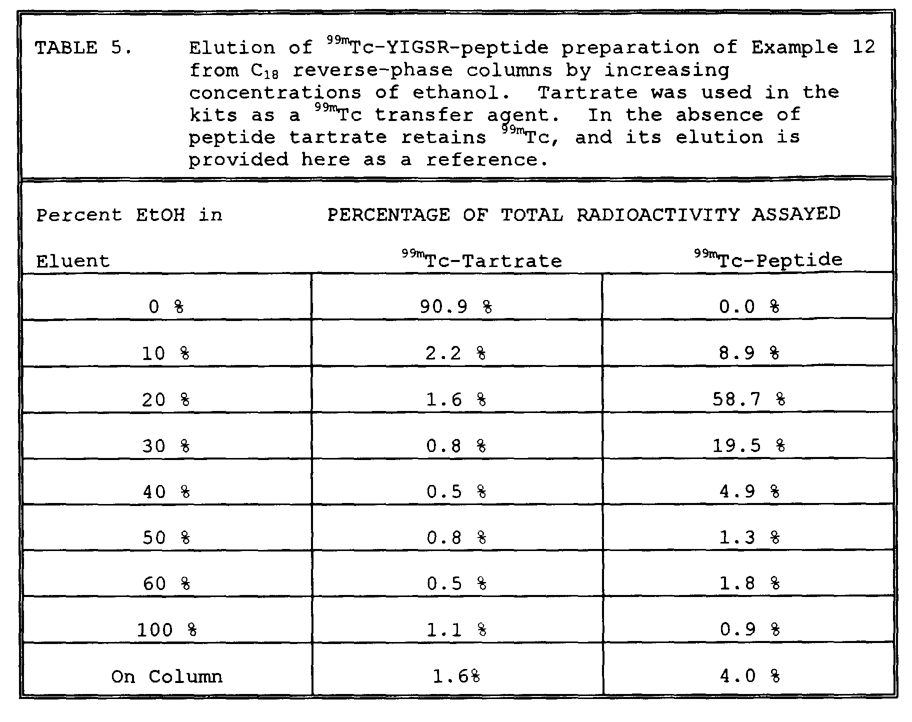

### EXAMPLE 15 - BIODISTRIBUTION OF ^{99m}Tc-YIGSR-CONTAINING PEPTIDE IN RODENTS

The biodistribution of the ^{99m}Tc-YIGSR-peptide of Example 12 was evaluated in adult female Swiss-Webster mice (approximately 19 g) at selected times (10, 30, and 120 minutes) after injection into the tail vein. Each experimental group was composed of at least five animals, with each animal receiving 0.1 ml containing 5 µg of peptide (1 µCi/µg). Animals were sacrificed by Halothane overdose, and selected organs dissected, weighed, and associated radioactivity determined. Data were analysed using a computer program specifically designed for ^{99m}Tc-labelled preparations. The percent dose per organ for blood, bone, and muscle were calculated assuming 7, 8.2, and 40% of total body weight, respectively, for these tissues.

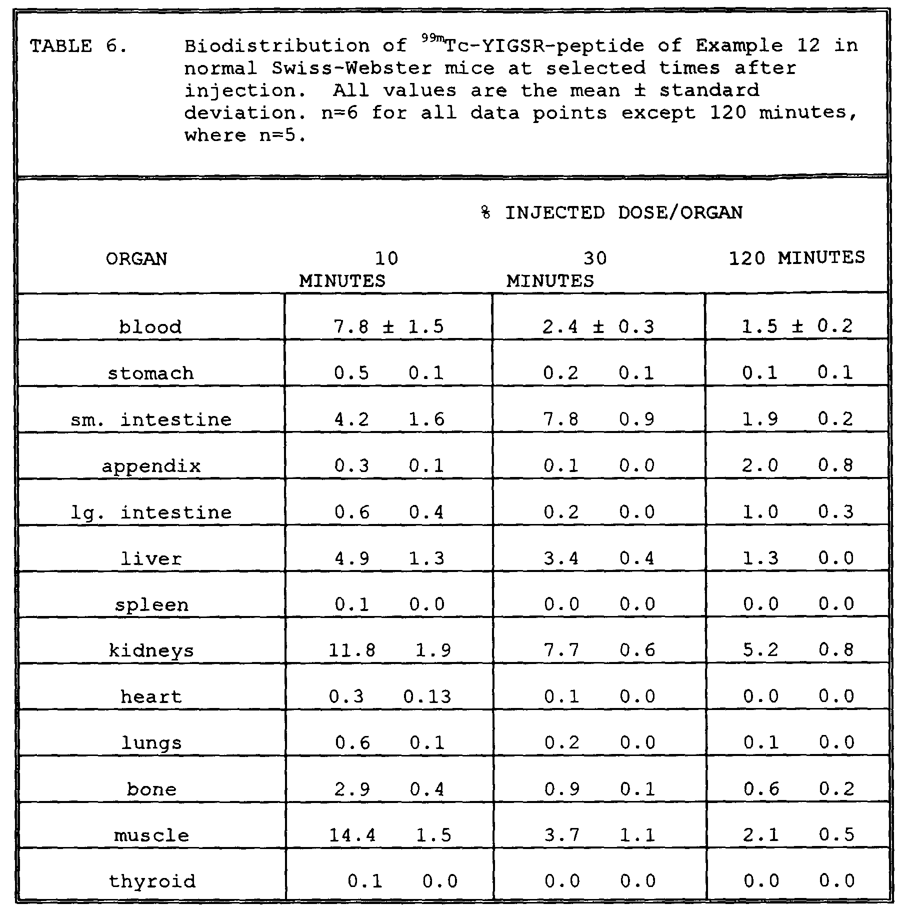

Some studies involved pre-incubation of the ^{99m}Tc-YIGSR-peptide in whole blood prior to injection and determination of biodistribution (Table 7). In these studies, whole human blood was obtained from a healthy adult male donor and collected into Vacutainer tubes containing EDTA. After mixing to insure proper dissolution of the EDTA, approximately 2.5 ml of the whole blood was removed and mixed with 0.25 ml of ^{99m}Tc-YIGSR-peptide. The mixture was allowed to incubate 30 minutes at room temperature. After 30 minutes, aliquots of 0.1 ml were injected into the tail vein of the mice. The amount of radioactivity in the circulation for ^{99m}Tc-YIGSR-peptide of Example 12 pre-incubated in whole blood was similar to that in animals receiving ^{99m}Tc-YIGSR-peptide without incubation in blood.

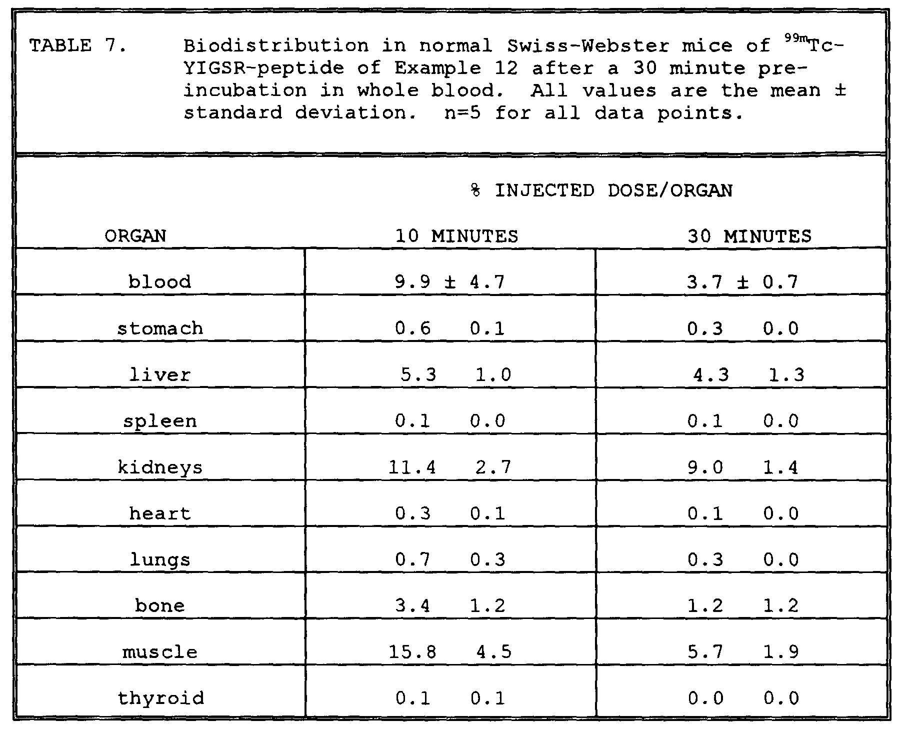

The clearance rates of the ^{99m}Tc-YIGSR-peptide of Example 12 was evaluated in adult female Sprague-Dawley rats at 2 hours after injection. Each experimental group was composed of three animals. Each animal was anaesthetised with ketamine and the bile duct and bladder were cannulated. Blood was collected over various periods of time from the jugular vein. The ^{99m}Tc-YIGSR-peptide cleared very rapidly from the plasma of rats, with a clearance rate of 2.6 ml/minute. At two hours, 31.2 ± 9.1% of the injected doses had cleared through urine, while bile clearance at the same time point was 6.9 ± 0.3%. In biodistribution studies, the highest amount of radioactivity was found in the kidneys, and is consistent with the rat clearance data. No major accumulation of radioactivity was found in any organ examined (other than kidney), and at later times (2 and 4 hours post injection) no re-distribution of the radiolabel was noted.

### EXAMPLE 16 - IN VITRO BINDING OF ^{99m}Tc-YIGSR-PEPTIDE TO PLATELETS AND COLON CARCINOMA CELLS

The YIGSR-containing peptide of Example 12, labelled with ^{99m}Tc as in Example 13, was used to measure relative binding to colon carcinoma cells, platelets and induced clots. In these studies, ^{99m}Tc-human IgG was used as a control. Measures were expressed as a percent of final counts per minute, using the control ^{99m}Tc-human IgG as 100%.

For studies of LS-174T binding, cells were grown in cell culture. For studies of binding to platelets and induced clots, normal whole human blood was collected in citrated buffer, and the platelet-rich plasma collected by differential centrifugation. Platelets were either used directly, or were clotted. For clot studies, 2 drops of a saturated solution of calcium chloride and magnesium chloride were added to 1 ml of platelet-rich plasma, clots were allowed to form, rinsed in buffer, and placed in phosphate buffered saline containing 1% bovine serum albumin. In all experiments, the ^{99m}Tc-YIGSR-peptide and ^{99m}Tc-human IgG preparations were allowed to incubate for 30 minutes at 37°C with the carcinoma cells, platelets and clots. For cells and platelets, separation preparatory to counting was by centrifugation; for clots, separation was by washing.

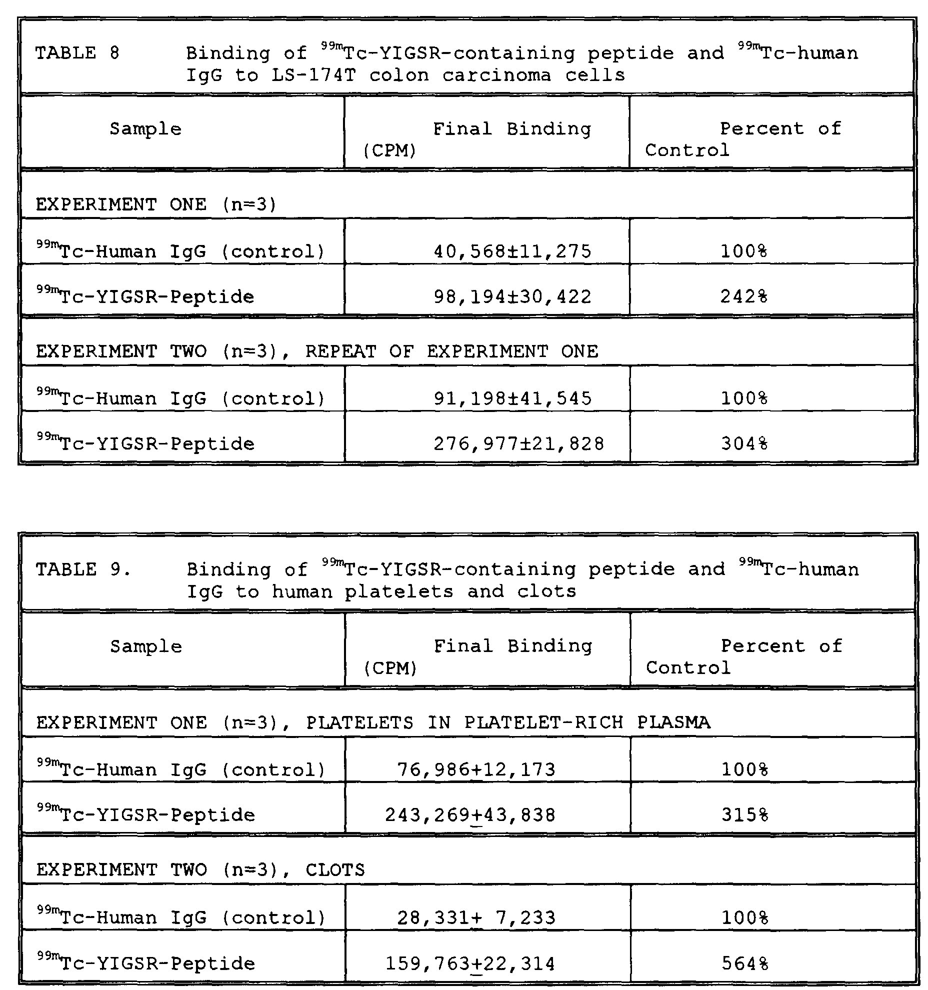

In other experiments, the peptide of Example 12, labelled as in Example 13, was used for in vitro binding studies. In these studies, the ^{99m}Tc-YIGSR-containing peptide was incubated for 90 minutes with either 0.5 ml of whole blood clots, using from 0.4 to 50 µg of radiolabelled peptide, or with approximately 10⁷ platelets, using from 2 to 200 µg of radiolabelled peptide. These studies showed a dose-response relationship between the amount of radiolabelled peptide and the blood clots or platelets.

### EXAMPLE 17 - IN VIVO LOCALISATION IN INDUCED CLOTS WITH ^{99m}Tc-YIGSR CONTAINING PEPTIDE

Experimental jugular thrombi were induced in adult Fisher 344 rats using 20 µg/0.1 ml of thrombin. Studies were conducted using quantitative whole body autoradiography. The YIGSR-containing peptide of Example 12, labelled with ^{99m}Tc as in Example 13, was injected intravenously, with whole body autoradiography conducted 90 minutes after injection. Whole body autoradiography showed rapid clearance of radioactivity through or by kidneys, and to a lesser degree, through the biliary system. Significant accumulation of radioactivity was noted in the induced jugular thrombi, with a thrombus to muscle ratio of 15:1.5, and a thrombus to blood ratio of 3:1.

### EXAMPLE 18 - MODIFIED YIGSR-CONTAINING PEPTIDE WITH MULTIPLE RECOGNITION UNITS

A peptide with a longer sequence to improve blood retention and repeated sequences of YIGSR to improve binding to platelets is synthesised. Synthesis is done by solid-phase synthesis techniques using t-butyloxycarbonyl (Boc) protected amino acids added sequentially to a Gly-resin ester, followed by reverse-phase HPLC purification. The sequence of the peptide is as follows:
CDGGG**YIGSR**GG**YIGSR**GGGDC
   (Cys-Asp-Gly-Gly-Gly-Tyr-Ile-Gly-Ser-Arg-Gly-Gly-Tyr-Ile-Gly-Ser-Gly-Gly-Gly-Arg-Cys)
The foregoing peptide has a purity of greater than 98% as determined by reverse phase HPLC. The amino acid composition is confirmed by amino acid analysis.

The foregoing peptide is dissolved directly in nitrogen-purged 10 mM/40 mM tartrate/phthalate buffer, pH 5.5 (P/T buffer). The dissolved YIGSR-containing peptide is adjusted to a final concentration of 1 mg/ml in 10 mM P/T buffer containing 40 µg/ml of stannous tartrate and stored frozen, under a nitrogen atmosphere, in 5 cc amber serum-vials until labelled. For labelling, a vial is allowed to come to room temperature and ^{99m}Tc, as sodium pertechnetate, is added. The labelling reaction is allowed to proceed for 30 minutes. Essentially all of the ^{99m}Tc is complexed to the peptide as determined by HPLC analysis.

### EXAMPLE 19 - MODIFIED YIGSR-CONTAINING PEPTIDE WITH MULTIPLE RECOGNITION UNITS AND D-AMINO ACID SEQUENCES

A peptide containing D-amino acid sequences is used to confer metabolic resistance for in vivo use. The peptide of Example 18 is modified to include such D-amino acid sequences. Synthesis is done by solid-phase synthesis techniques using t-butyloxycarbonyl (Boc) protected amino acids added sequentially to a Gly-resin ester, followed by reverse-phase HPLC purification. The sequence of the peptide is as follows:
(D)-Cys-(D)-Asp-Gly-Gly-Gly-(D)-Tyr-Ile-Gly-(D)-Ser-Arg-Gly-Gly-(D)-Tyr-Ile-Gly-(D)-Ser-Gly-Gly-Gly-Asp-(D)-Cys
The forgoing peptide has a purity of greater than 98% as determined by reverse phase HPLC. The amino acid composition is confirmed by amino acid analysis.

The foregoing peptide is dissolved directly in nitrogen-purged 10 mM/40 mM tartrate/phthalate buffer, pH 5.5 (P/T buffer). The dissolved YIGSR-containing peptide is adjusted to a final concentration of 1 mg/ml in 10 mM P/T buffer containing 40 µg/ml of stannous tartrate and stored frozen, under a nitrogen atmosphere, in 5 cc amber serum-vials until labelled. For labelling, a vial is allowed to come to room temperature and ^{99m}Tc, as sodium pertechnetate, is added. The labelling reaction is allowed to proceed for 30 minutes. Essentially all of the ^{99m}Tc is complexed to the peptide as determined by HPLC analysis.

### EXAMPLE 20 - PREPARATION OF LYOPHILISED YIGSR-CONTAINING PEPTIDE RADIOLABELLING KITS

YIGSR-containing peptide radiolabelling kits of Examples 12, 18 or 19 are prepared, with the addition of glycine and inositol as excipients. The kits are then individually vialed and lyophilised.

### EXAMPLE 21 - ANIMAL LOCALISATION STUDIES USING ^{99m}Tc-YIGSR-CONTAINING PEPTIDES

The YIGSR-containing peptide kits of Examples 12, 18, 20, or 20 are used in animal localisation studies of induced pulmonary thromboembolism in adult Swiss-Webster mice with collagen/adrenaline-induced pulmonary embolism. Immediately prior to use in the studies, the animals are anaesthetised by an intramuscular injection of pentobarbital. Each mouse is injected with 0.1 ml of saline containing 10 µg of collagen and 5 µg of adrenaline. This treatment results in the aggregation of circulating platelets and the subsequent lung deposition of emboli. Animals so treated exhibit 20-30% thrombocytopenia relative to control animals. Control animals receive sham injections of 0.1 ml saline. After an appropriate amount of time to allow for the development of pulmonary thromboembolism (5-15 minutes), the animals are injected with ^{99m}Tc-YIGSR-containing peptides of Examples 12, 18, 19, or 20, and 10 and 30 minute biodistribution studies are performed.

### EXAMPLE 22 - DIAGNOSTIC IMAGING OF THROMBOSIS USING ^{99m}Tc-YIGSR-CONTAINING PEPTIDE KITS

A kit of Examples 12, 18, 19, or 20 is used in to localise thromboembolism in a patient. After radiolabelling with ^{99m}Tc as in Example 13, the ^{99m}Tc-YIGSR-peptide is injected intravenously. Starting immediately upon injection, the patient is imaged, using conventional gamma scintigraphy or SPECT imaging, and is imaged at 30 minute intervals thereafter. Sites of thromboembolism will appear as photon-rich image locations by scintigraphy consistent with circulatory distribution.

### EXAMPLE 23 - OTHER RADIOLABELLED PEPTIDES

In addition to the specific examples above, the methods of this invention have been successfully applied to the following peptides:
a) angiotensin I,
b) renin substrate tetradecapeptide,
c) hypercalcemia of malignancy factor fragment 1-16,
d) parathyroid hormone fragment 1-34,
e) poly(histidine-glutamic acid)-poly-alanine-poly-lysine, and
f) additional chemotactic peptide analogs.

## Claims

1. A method of preparing a pharmaceutical composition containing a labelled peptide suitable for administration to a patient, characterised by:
(a) obtaining or preparing a peptide substrate comprising a biological function domain (BD) and a medically useful labelling metal ion binding domain (MD), said substrate having a formula selected from:
(R₁) - [Y₁]ₙ - (R₂₎,
(R₁) - [Y₁ - (R₂) - Y₁]ₙ - (R₃) and
(R₁) - [Y₁ - (R₂) - Y₂]ₙ - (R₃)
where
R₁, R₂ and R₃ each comprise an amino acid sequence containing from 0 to 20 amino acids,
Y₁ and Y₂ are amino acids containing S N and/or O capable of complexing with ions of a transition metal selected from Zn, Cu, Sn, Co and Ni, and
n is an integer from 1 to 6
wherein
said MD is selected from [Y₁]ₙ, [Y₁ - (R₂) - Y₁]ₙ and [Y₁ - (R₂) - Y₂]ₙ and
said BD comprises at least one of R₁, R₂ and R₃ and includes an amino acid sequence containing from 1 to 20 amino acids.
(b) reacting said substrate in a pharmaceutically acceptable aqueous buffer solution with a source of ions of a said transition metal so as to form complexes of said S, N and/or O-containing amino acid and said transition metal ions, and
(c) reacting said complexed substrate in said buffer solution with a medically useful labelling metal ion, said labelling ion having a higher order of binding than said transition metal ion so as to replace the transition metal ion by said labelling metal ion on said MD, thereby producing said composition comprising a labelled peptide in said buffer,
provided that where said substrate includes disulfide bonds these are first reduced by incubation of the substrate with a reducing agent and excess reducing agent is afterwards removed.

2. A method according to claim 1 wherein said MD includes one or more amino acid sequences containing monosulfide groups without disulfide bonds.

3. A method according to claim 1 or claim 2 wherein said MD includes an amino acid sequence selected from cysteine, histidine, penicillamine, deacylated methionine, lysine, arginine, aspartic acid, glutamic acid and tyrosine.

4. A method according to any preceding claim wherein said MD is selected from
[Cys - (R₂) - Cys]ₙ
[Cys - (R₂) - Pen]ₙ
[His - (R₂) - Cys]ₙ
[His - (R₂) - Pen]ₙ
[His]ₙ and
[His - (R₂) - His]ₙ
where n is from 1 to 6 and R₂ is an amino acid sequence containing from 1 to 20 amino acids.

5. A method according to any preceding claim wherein said BD is selected from
Phe - Trp - Lys - Thr,
N-formyl - Met - Leu - Phe,
Tyr - Ile - Gly - Ser - Arg (YIGSR) and
Ile - Lys - Val - Ala - Val (IKVAV)

6. A method according to claim 4 or claim 5 wherein the peptide contains a sequence selected from
Arg - Lys - Gln - Ala - Ala - Ser- Ile - Lys - Val - Ala - Val,
(RKQAASIKVAV)
Cys-Ser-Arg-Ala-Arg-Lys-Gln-Ala-Ala-Ser-Ile-Lys-Val-Ala-Val-Ser-Ala-Asp-Arg,
(CSRARKQAASIKVAVSADR) and
Cys - Asp - Pro - Gly - Tyr - Ile - Gly - Ser - Arg
(CDPGYIGSR)

7. A method according to any preceding claim wherein said transition metal ion is stannous (Sn (II).

8. A method according to claim 7 wherein the source of said stannous ions comprises a member selected from stannous tartrate, stannous glucoheptonate, stannous gluconate, stannous phosphonate, stannous chloride and stannous fluoride.

9. A method according to any preceding claim wherein said labelling metal ion is Tc and during step (c) a reducing agent is added to reduce the oxidation state of said labelling metal ion.

10. A method according to claim 6 or claim 7 wherein during step (c) a reducing agent is added to reduce the oxidation state of said labelling metal ion and wherein said source of stannous ions and said reducing agent each consist essentially of stannous tartrate.

11. A method according to any preceding claim wherein the said buffer solution comprises anions of one or more dicarboxylic acids.

12. A method according to claim 11 wherein said dicarboxylic anions(s) are selected from phthalate, tartrate and citrate.

13. A method according to any preceding claim wherein said transition metal ion is stannous and the said buffer solution includes alkali metal tartrate at a pH from 5 to 6.

14. A method according to any preceding claim wherein said buffer solution consists essentially of a mixture of 10 mM tartrate and 40 mM phthalate.

15. A method according to any of claims 11 to 14 wherein said buffer also contains an amino acid.

16. A method according to claim 15 wherein said amino acid is glycine.

17. A method according to any preceding claim wherein the said labelling ion is selected from ions of Fe, Co, Ni, Cu, Zn, As, Se, Mo, Tc, Ru, Pd, Ag, Cd, In, Re, Os, Ir, Pt, Au, Hg, Tl, Pb, Bi, Po and At.

18. A method according to any preceding claim wherein the said labelling ion is selected from radioactive ions of Tc, Re, Cu, Ag, Hg, Au and In.

19. A method according to any preceding claim wherein the composition prior to step (c) is stored in dosage vials in lyophilised form and rehydrated during or immediately prior to step (c).

20. A pharmaceutical composition containing a labelled peptide as defined in claim 1 suitable for administration to a patient, said peptide comprising a biological function domain (BD) and a medically useful labelling metal ion binding domain (MD) comprising at least one amino acid containing sulfur, nitrogen and/or oxygen capable of complexing with ions of a transition metal selected from Zn, Cu, Sn, Co and Ni ,, and a medically useful labelling metal ion,
characterised in that:
(a) said MD is as defined in any one of claims 1 to 4, and
(b) said BD comprises one of:
Tyr - Ile - Gly - Ser - Arg (YIGSR) and
Ile - Lys - Val - Ala - Val (IKVAV)
(c) said labelling metal ion is directly coupled to said sulfur, nitrogen and/or oxygen of said MD.

21. A composition according to claim 20 wherein the peptide contains one or more sequences selected from
Arg - Lys - Gln - Ala - Ala - Ser- Ile - Lys - Val - Ala - Val,
(RKQAASIKVAV)
Cys-Ser-Arg-Ala-Arg-Lys-Gln-Ala-Ala-Ser-Ile-Lys-Val-Ala-Val-Ser-Ala-Asp-Arg,
(CSRARKQAASIKVAVSADR) and
Cys - Asp - Pro - Gly - Tyr - Ile - Gly - Ser - Arg
(CDPGYIGSR)

22. A composition according to claim 20 or claim 21 wherein said labelling metal ion is as defined in claim 17 or claim 18.

23. A composition according to any one of claims 20 to 22 wherein the peptide is present in an aqueous buffer as defined in any one of claims 11 to 16.

24. A lyophilised or frozen dosage unit containing a targeting agent suitable for administration to a patient and ready (upon rehydration) for direct labelling by the addition of radioactive pertechnetate or perhenate,
characterised in that said agent comprises a peptide as defined in claim 1 comprising a biological function domain (BD) and a medically useful labelling metal ion binding domain (MD), wherein
(a) said MD is as defined in any one of claims 2 to 4, and
(b) said BD comprises one of:
Tyr - Ile - Gly - Ser - Arg (YIGSR) and
Ile - Lys - Val - Ala - Val (IKVAV)

25. A dosage unit according to claim 24 wherein said peptide contains one or more sequences selected from
Arg - Lys - Gln - Ala - Ala - Ser- Ile - Lys - Val - Ala - Val,
(RKQAASIKVAV)
Cys-Ser-Arg-Ala-Arg-Lys-Gln-Ala-Ala-Ser-Ile-Lys-Val-Ala-Val-Ser-Ala-Asp-Arg,
(CSRARKQAASIKVAVSADR) and
Cys - Asp - Pro - Gly - Tyr - Ile - Gly - Ser - Arg
(CDPGYIGSR)

26. A dosage unit according to claims 24 or claim 25 wherein the peptide is present in an aqueous buffer as defined in any one of claims 11 to 16.

27. A composition in dosage form containing a targeting agent suitable for administration to a patient consisting of rehydrated contents of a dosage unit according to any one of claims 24 to 26 which have been labelled by the addition of a radioactive labelling metal ion.

28. A composition according to claim 27 wherein said labelling metal ion is radioactive pertechnetate.

29. The use of a peptide as claimed in claim 5 or claim 6 for the preparation of a composition according to any one of claims 20 to 23 or 28 or 29, or of a dosage unit according to any one of claim 24 to 26 for administration to a patient for diagnostic imaging of the lung, wherein said BD comprises the sequence
Ile - Lys - Val - Ala - Val (IKVAV).

30. The use according to claim 29 wherein the peptide contains the sequence
Arg - Lys - Gln - Ala - Ala - Ser- Ile - Lys - Val - Ala - Val,
(RKQAASIKVAV) or
Cys-Ser-Arg-Ala-Arg-Lys-Gln-Ala-Ala-Ser-Ile-Lys-Val-Ala-Val-Ser-Ala-Asp-Arg,
(CSRARKQAASIKVAVSADR)

31. The use according to claim 30 wherein the peptide consists essentially of the sequence
Cys-Ser-Arg-Ala-Arg-Lys-Gln-Ala-Ala-Ser-Ile-Lys-Val-Ala-Val-Ser-Ala-Asp-Arg.
(CSRARKQAASIKVAVSADR)
including repetitions of IKVAV.

32. The use of a peptide as claimed in claim 5 or claim 6 for the preparation of a composition according to any one of claims 20 to 23 or 27 or 28, or of a dosage unit according to any one of claim 24 to 26 for administration to a patient for the detection of sites of platelet accumulation or carcinomas, wherein said BD comprises the sequence
Tyr - Ile - Gly - Ser - Arg (YIGSR)

33. The use according to claim 32 wherein the peptide also contains the sequence
Asp - Pro - Gly

34. The use according to claim 33 wherein the peptide consists essentially of the sequence
Cys - Asp - Pro - Gly - Tyr - Ile - Gly - Ser - Arg
(CDPGYIGSR)
including repetitions of YIGSR.

35. The use of a peptide as defined in claim 1 containing thiolate groups complexed through the sulfur atoms of the thiolate groups with ions of a transition metal selected from Zn, Cu, Sn, Co and Ni, for direct labelling by reaction of the complexed peptide in a pharmaceutically acceptable buffer with a medically useful labelling metal ion having a higher order of binding than said transition metal ion, whereby said labelling metal ions replace said complexed transition metal ions resulting in a composition comprising a labelled peptide in said buffer.

36. The use according to claim 35 wherein said transition metal ions are stannous ions.

## Patentansprüche

1. Methode zur Herstellung einer pharmazeutischen Zusammensetzung enthaltend ein markiertes Peptid, geeignet für die Verabreichung an einen Patienten, dadurch gekennzeichnet, dass man
(a) ein Peptidsubstrat vorlegt oder herstellt, welches einen biologischen Funktionsbereich (BD) und einen medizinisch nützlichen Markierungsmetallion-Bindungsbereich (MD) enthält, und dieses Substrat einer Formel entspricht, die ausgewählt ist aus:
(R₁) - [Y₁]ₙ - (R₂),
(R₁) - [Y₁ - (R₂) - Y₁]ₙ - (R₃) und
(R₁) - [Y₁ - (R₂) - Y₂]ₙ - (R₃),
worin
R₁, R₂ und R₃ unabhängig voneinander eine Aminosäurensequenz enthaltend 0 bis 20 Aminosäuren, bedeuten,
Y₁ und Y₂ Aminosäuren enthaltend S N, und/oder O, bedeuten, welche befähigt sind, mit Ionen eines Übergangsmetalls ausgewählt aus Zn, Cu, Sn, Co und Ni, zu komplexieren, und
n eine ganze Zahl von 1 bis 6 bedeutet,
worin
der genannte MD ausgewählt ist aus [Y₁]ₙ, [Y₁ - (R₂) - Y₁]ₙ und [Y₁ - (R₂) - Y₂]ₙ, und
der genannte BD mindestens einen Rest, ausgewählt aus R₁, R₂ und R₃ enthält, und eine Aminosäurensequenz mit 1 bis 20 Aminosäuren beinhaltet,
(b) dieses Substrat in einer pharmazeutisch annehmbaren wässerigen Pufferlösung, enthaltend einen Spender von Ionen der genannten Übergangsmetalle, zur Reaktion bringt, so dass sich Komplexe dieser S, N und/oder O enthaltenden Aminosäure mit den genannten Übergangsmetallionen bilden, und
(c) dieses komplexierte Substrat in dieser Pufferlösung mit einem medizinisch nützlichen markierenden Metallion zur Reaktion bringt, wobei dieses markierende Metallion einen höheren Bindungsgrad besitzt als das genannte Übergangsmetallion, so dass das an den MD gebundene Übergangsmetallion durch das genannte markierende Metallion ersetzt und in dieser Pufferlösung die genannte Zusammensetzung, enthaltend ein markiertes Peptid, gebildet wird,
wobei, wenn das Substrat Disulfidbindungen enthält, diese vorgängig durch Inkubation des Substrats mit einem Reduktionsmittel reduziert werden und ein Überschuss an Reduktionsmittel anschliessend entfernt wird.

2. Methode nach Anspruch 1, worin der genannte MD eine oder mehrere Aminosäurensequenzen umfasst, welche Monosulfidgruppen ohne Disulfidbindungen enthalten.

3. Methode nach Anspruch 1 oder Anspruch 2, worin der genannte MD eine Aminosäurensequenz enthält, ausgewählt aus Cystein, Histidin, Penicillamin, deacyliertem Methionin, Lysin, Arginin, Aspartinsäure, Glutaminsäure und Tyrosin.

4. Methode nach einem der voran gehenden Ansprüche, worin der genannte MD ausgewählt ist aus
[Cys - (R₂) - Cys]ₙ
[Cys - (R₂) - Pen]ₙ
[His - (R₂) - Cys]ₙ
[His - (R₂) - Pen]ₙ
[His]ₙ und
[His - (R₂) - His]ₙ
worin n einen Wert von 1 bis 6 und R₂ eine Aminosäurensequenz enthaltend 1 bis 20 Aminosäuren, bedeuten.

5. Methode nach einem der voran gehenden Ansprüche, worin der genannte BD ausgewählt ist aus
Phe - Trp - Lys - Thr,
N-Formyl - Met - Leu - Phe,
Tyr - Ile - Gly - Ser - Arg (YIGSR) und
Ile - Lys - Val- Ala - Val (IKVAV)

6. Methode nach Anspruch 4 oder Anspruch 5, worin das Peptid eine Sequenz enthält, die ausgewählt ist aus
Arg - Lys - Gln - Ala - Ala - Ser - Ile - Lys - Val - Ala - Val
(RKQAASIKVAV)
Cys-Ser-Arg-Ala-Arg-Lys-Gln-Ala-Ala-Ser-Ile-Lys-Val-Ala-Val-Ser-Ala-Asp-Arg
(CSRARKQAASIKVAVSADR) und
Cys - Asp - Pro - Gly - Tyr - Ile - Gly -Ser -Arg
(CDPGYIGSR).

7. Methode nach einem der voran gehenden Ansprüche, worin das genannte Übergangsmetallion ein Zinn Sn (II) ist.

8. Methode nach Anspruch 7, worin der Spender dieser Zinn-Ionen eine Verbindung beinhaltet, ausgewählt ist aus Zinntartrat, Zinnglucoheptonat, Zinngluconat, Zinnphosphonat, Zinnchlorid und Zinnfluorid.

9. Methode nach einem der voran gehenden Ansprüche, worin das markierende Metallion Tc ist und dass bei Durchführung der Stufe (c) ein Reduktionsmittel zugegeben wird, um den Oxidationszustand dieses markierenden Metallions zu reduzieren.

10. Methode nach Anspruch 6 oder Anspruch 7, worin bei Durchführung der Stufe (c) ein Reduktionsmittel zugefügt wird, um den Oxidationszustand des markierenden Metallions zu reduzieren, und worin der Spender der Zinn-Ionen und das Reduktionsmittel, beide jeweils im wesentlichen aus Zinntartrat bestehen.

11. Methode nach einem der voran gehenden Ansprüche, worin die genannte Pufferlösung Anionen von einer oder mehreren Dicarbonsäuren enthält.

12. Methode nach Anspruch 11, worin das genannte Dicarbonsäureanion bzw. Anionen ausgewählt sind von Phtalat-, Tartrat- und Citratanionen.

13. Methode nach einem der voran gehenden Ansprüche, worin das Übergangsmetallion Zinn darstellt und die Pufferlösung Alkalimetalltartrat bei einem pH von 5 bis 6, enthält.

14. Methode nach einem der voran gehenden Ansprüche, worin die Pufferlösung im wesentlichen aus einer Mischung von 10 mM Tartrat und 40 mM Phthalat besteht.

15. Methode nach einem der Ansprüche 11 bis 14, worin der genannte Puffer zusätzlich eine Aminosäure enthält.

16. Methode nach Anspruch 15, worin diese Aminosäure Glycin ist.

17. Methode nach einem der voran gehenden Ansprüche, worin das markierende Ion ausgewählt ist aus den Ionen Fe, Co, Ni, Cu, Zn, As, Se, Mo, Tc, Ru, Pd, Ag, Cd, In, Re, Os, Ir, Pt, Au, Hg, Tl, Pb, Bi, Po, und At.

18. Methode nach einem der voran gehenden Ansprüche, worin das markierende Ion ausgewählt ist aus radioaktiven Ionen von Tc, Re, Cu, Ag, Hg, Au und In.

19. Methode nach einem der voran gehenden Ansprüche, worin die Zusammensetzung vorgängig zu Stufe (c) in Dosierungsbehältern in lyophilisierter Form gelagert wird und während oder direkt vor der Stufe (c) rehydratisiert wird.

20. Pharmazeutische Zusammensetzung enthaltend ein markiertes Peptid gemäss Anspruch 1, geeignet für die Verabreichung an einen Patienten, wobei dieses Peptid einen biologischen Funktionsbereich (BD) und einen medizinisch nützlichen Markierungsmetallion-Bindungsbereich (MD) enthält, welcher mindestens eine Schwefel, Stickstoff und/oder Sauerstoff enthaltende Aminosäure aufweist, welche befähigt ist, mit Ionen eines Übergangsmetalls, ausgewählt aus Zn, Cu, Sn, Co und Ni, zu komplexieren, und ein medizinisch nützliches markierendes Metallion, dadurch gekennzeichnet, dass
(a) der genannte MD gemäss einem der Ansprüche 1 bis 4 definiert ist, und
(b) der genannte BD eine der Sequenzen von
Tyr - Ile - Gly - Ser - Arg (YIGSR) und
Ile - Lys - Val- Ala - Val (IKVAV)
enthält,
(c) das genannte markierende Metallion direkt mit dem genannten Schwefel, Stickstoff und/oder Sauerstoff des MD verbunden ist.

21. Zusammensetzung nach Anspruch 20, worin das Peptid eine oder mehrere Sequenzen enthält, welche ausgewählt sind aus
Arg - Lys - Gln - Ala - Ala - Ser - Ile - Lys - Val - Ala - Val
(RKQAASIKVAV)
Cys-Ser-Arg-Ala-Arg-Lys-Gln-Ala-Ala-Ser-Ile-Lys-Val-Ala-Val-Ser-Ala-Asp-Arg
(CSRARKQAASIKVAVSADR) und
Cys - Asp - Pro - Gly - Tyr - Ile - Gly -Ser -Arg
(CDPGYIGSR).

22. Zusammensetzung nach Anspruch 20 oder Anspruch 21, worin das markierende Metallion der Definition von Anspruch 17 oder Anspruch 18 entspricht.

23. Zusammensetzung nach einem der Ansprüche 20 bis 22, worin das Peptid in einer wässerigen Pufferlösung, gemäss einem der Ansprüche 11 bis 16, anwesend ist.

24. Lyophilisierte oder gefrorene Dosierungseinheit enthaltend einen Zielwirkstoff geeignet für die Verabreichung an einen Patienten und (nach Rehydratation) bereit ist für die direkte Markierung durch Zugabe von radioaktivem Pertechnetat oder Perhenat, dadurch gekennzeichnet, dass dieser Wirkstoff ein Peptid gemäss der Definition von Anspruch 1 enthält, und einen biologischen Funktionsbereich (BD) und einen medizinisch nützlichen Markierungsmetallion-Bindungsbereich (MD) aufweist, worin
(a) der genannte MD gemäss einem der Ansprüche 2 bis 4 definiert ist, und
(c) der genannte BD eine der Sequenzen
Tyr - Ile - Gly - Ser - Arg (YIGSR) und
Ile - Lys - Val- Ala - Val (IKVAV)
enthält.

25. Dosierungseinheit nach Anspruch 24, worin das genannte Peptid eine oder mehrere Sequenzen enthält, welche ausgewählt sind aus
Arg - Lys - Gln - Ala - Ala - Ser - Ile - Lys - Val - Ala - Val
(RKQAASIKVAV)
Cys-Ser-Arg-Ala-Arg-Lys-Gln-Ala-Ala-Ser-Ile-Lys-Val-Ala-Val-Ser-Ala-Asp-Arg
(CSRARKQAASIKVAVSADR) und
Cys - Asp - Pro - Gly - Tyr - Ile - Gly -Ser -Arg
(CDPGYIGSR).

26. Dosierungseinheit nach Anspruch 24 oder Anspruch 25, worin das Peptid in einem wässerigen Puffer anwesend ist, wie in einem der Ansprüche 11 bis 16 definiert.

27. Zusammensetzung in dosierter Form enthaltend einen Zielwirkstoff geeignet für die Verabreichung an einen Patienten bestehend aus rehydratisierten Inhalten einer Dosierungseinheit gemäss einem der Ansprüche 24 bis 26, welche durch die Zugabe eines radioaktiven Markierungsmetallions markiert wurden.

28. Zusammensetzung nach Anspruch 27, worin das Markierungsmetallion radioaktives Pertechnetat darstellt.

29. Verwendung eines Peptids gemäss Anspruch 5 oder Anspruch 6 für die Herstellung einer Zusammensetzung gemäss einem der Ansprüche 20 bis 23 oder 28 oder 29, oder einer Dosierungseinheit nach einem der Ansprüche 24 bis 26 für die Verabreichung an einen Patienten für die diagnostische Abbildung der Lunge, worin der genannte BD die Sequenz
Ile - Lys - Val- Ala - Val (IKVAV)
enthält.

30. Verwendung nach Anspruch 29, worin das Peptid die Sequenz
Arg - Lys - Gln - Ala - Ala - Ser - Ile - Lys - Val - Ala - Val
(RKQAASIKVAV) oder
Cys-Ser-Arg-Ala-Arg-Lys-Gln-Ala-Ala-Ser-Ile-Lys-Val-Ala-Val-Ser-Ala-Asp-Arg
(CSRARKQAASIKVAVSADR)
enthält.

31. Verwendung nach Anspruch 30, worin das Peptid im wesentlichen aus der Sequenz
Cys-Ser-Arg-Ala-Arg-Lys-Gln-Ala-Ala-Ser-Ile-Lys-Val-Ala-Val-Ser-Ala-Asp-Arg
(CSRARKQAASIKVAVSADR)
einschliesslich Wiederholungen von IKVAV, besteht.

32. Verwendung eines Peptids gemäss Anspruch 5 oder Anspruch 6 für die Herstellung einer Zusammensetzung gemäss einem der Ansprüche 20 bis 23 oder 27 oder 28, oder einer Dosierungseinheit nach einem der Ansprüche 24 bis 26 für die Verabreichung an einen Patienten für die Feststellung von Stellen von Anhäufung von Blutplättchen oder von Karzinomen, worin der genannte BD die Sequenz
Tyr - Ile - Gly - Ser - Arg (YIGSR)
enthält.

33. Verwendung nach Anspruch 32, worin das Petid auch die Sequenz Asp - Pro - Gly enthält.

34. Verwendung nach Anspruch 33, worin das Peptid im wesentlichen aus der Sequenz
Cys - Asp - Pro - Gly - Tyr - Ile - Gly -Ser -Arg
(CDPGYIGSR)
einschliesslich Wiederholungen von YIGSR besteht.

35. Verwendung eines Pepides gemäss Anspruch 1, enthaltend Thiolatgruppen, welche über das Schwefelatom der Thiolatgruppen komplexiert sind mit Ionen eines Übergangmetalles ausgewählt von Zn, Cu, Sn, Co und Ni, zwecks direkter Markierung durch Umsetzung des komplexierten Peptides in einem pharmazeutisch annehmbaren Puffer mit einem medizinisch nützlichen markierenden Metallion, welches einen höheren Bindungsgrad besitzt als das genannte Übergangsmetallion, so dass das Übergangsmetallion durch das genannte markierende Metallion ersetzt wird und eine Zusammensetzung entsteht, welche ein markiertes Peptid in der genannten Pufferlösung enthält.

36. Verwendung nach Anspruch 35, worin diese Übergangsmetallionen Zinnionen sind.

## Revendications

1. Une méthode de préparation d'une composition pharmaceutique contenant un peptide marqué, utilisable pour l'administration à un patient, caractérisée en :
(a) l'obtention ou la préparation d'un substrat de peptide comprenant un domaine de fonction biologique (BD) et un domaine de liaison d'ion de métal marquant médicalement utile (MD), ledit substrat ayant une formule choisie parmi :
(R₁) - [Y₁]ₙ - (R₂),
(R₁) - [Y₁ - (R₂) - Y₁]ₙ - (R₃) et
(R₁) - [Y₁ - (R₂) - Y₂]ₙ - (R₃),
où
R₁, R₂ et R₃ comprenant chacun une séquence d'acide aminé contenant entre 0 et 20 acides aminés,
Y₁ et Y₂ sont des acides aminés contenant S, N et/ ou O, capable de complexer avec des ions d'un métal de transition choisi dans le groupe comprenant Zn, Cu, Sn, Co et Ni, et
n est un nombre entier compris entre 1 et 6
où
ledit MD est choisi parmi [Y₁]ₙ, [Y₁ - (R₂) - Y₁]ₙ et [Y₁ - (R₂) - Y₂]ₙ et
ledit BD comprend au moins l'un parmi (R₁), (R₂) et (R₃)et inclut une séquence d'acide aminé contenant de 1 à 20 acides aminés.
(b) la réaction dudit substrat dans une solution tampon aqueuse pharmaceutiquement acceptable avec une source d'ions d'un dit métal de transition afin de former des complexes dudit acide aminé contenant du S, N et/ ou O et desdits ions de métal de transition, et
(c) la réaction dudit substrat complexé dans ladite solution tampon avec un ion de métal marquant médicalement utile, ledit ion marquant ayant un ordre de liaison plus élevé que ledit ion de métal de transition afin de remplacer l'ion de métal de transition par ledit ion de métal marquant dans ledit MD, produisant ainsi ladite composition comprenant un peptide marqué dans ledit tampon,
à condition que dans le cas où ledit substrat inclue des ponts disulfures, ceux-ci sont d'abord réduits par incubation du substrat avec un agent réducteur et l'excédent d'agent réducteur est ensuite enlevé.

2. Une méthode selon la revendication 1, où ledit MD inclut un ou plusieures séquences d'acide aminé contenant des groupes monosulfures sans ponts disulfures.

3. Une méthode selon la revendication 1 ou 2, où ledit MD inclut une séquence d'acide aminé choisie parmi cystéine, histidine, pénicillamine, méthionine déacylé, lysine, arginine, acide aspartique, acide glutamique et tyrosine.

4. Une méthode selon l'une quelconque des revendications précédentes, où ledit MD est choisi parmi
[Cys - (R₂) - Cys]ₙ
[Cys - (R₂) - Pen]ₙ
[His - (R₂) - Cys]ₙ
[His - (R₂) - Pen]ₙ
[His]ₙ et
[His - (R₂) - His]ₙ
où n est compris entre 1 et 6 et R₂ est une séquence d'acide aminé contenant entre 1 et 20 acides aminés.

5. Une méthode selon l'une quelconque des revendications précédentes, où ledit BD est choisi parmi
Phe - Trp - Lys - Thr,
N-formyl - Met - Leu - Phe,
Tyr - Ile - Gly - Ser - Arg (YIGSR) et
Ile - Lys - Val- Ala - Val (IKVAV)

6. Une méthode selon la revendication 4 ou la revendication 5, où le peptide contient une séquence choisie parmi
Arg - Lys - Gln - Ala - Ala - Ser - Ile - Lys - Val - Ala - Val
(RKQAASIKVAV)
Cys-Ser-Arg-Ala-Arg-Lys-Gln-Ala-Ala-Ser-Ile-Lys-Val-Ala-Val-Ser-Ala-Asp-Arg
(CSRARKQAASIKVAVSADR) et
Cys - Asp - Pro - Gly - Tyr - Ile - Gly -Ser -Arg
(CDPGYIGSR)

7. Une méthode selon l'une quelconque des revendications précédentes, où ledit ion de métal de transition est de l'étain (Sn(II).

8. Une méthode selon la revendication 7, où la source desdits ions d'étain comprend un membre choisi parmi tartrate stanneux, glucoheptonate stanneux, gluconate stanneux, phosphonate stanneux, chlorure stanneux et fluorure stanneux.

9. Une méthode selon l'une quelconque des revendications précédentes, où ledit ion de métal marquant est Tc et où durant l'étape (c) est ajouté un agent réducteur afin de réduire la phase d'oxydation dudit ion de métal marquant.

10. Une méthode selon la revendication 6 ou la revendication 7, où durant l'étape (c) est ajouté un agent réducteur afin de réduire la phase d'oxydation dudit ion de métal marquant et où ladite source d'ions d'étain et ledit agent réducteur consistent chacun essentiellement en tartrate stanneux.

11. Une méthode selon l'une quelconque des revendications précédentes, où ladite solution tampon comprend des anions d'un ou de plusieurs acides dicarboxyliques.

12. Une méthode selon la revendication 11, où ledit anion dicarboxylique resp. lesdits anions dicarboxyliques sont choisis parmi phtalate, tartrate et citrate.

13. Une méthode selon l'une quelconque des revendications précédentes, où ledit ion de métal de transition est de l'étain et ladite solution tampon contient du tartrate de métal alcalin à une valeur pH comprise entre 5 et 6.

14. Une méthode selon l'une quelconque des revendications précédentes, où ladite solution tampon consiste essentiellement en un mélange de 10 mM de tartrate et 40 mM de phtalate.

15. Une méthode selon l'une quelconque des revendications 11 à 14, où ledit tampon contient également un acide aminé.

16. Une méthode selon la revendication 15, où ledit acide aminé est glycine.

17. Une méthode selon l'une quelconque des revendications précédentes, où ledit ion marquant est choisi dans le groupe d'ions comprenant Fe, Co, Ni, Cu, Zn, As, Se, Mo, Tc, Ru, Pd, Ag, Cd, In, Re, Os, Ir, Pt, Au, Hg, Tl, Pb, Bi, Po, et At.

18. Une méthode selon l'une quelconque des revendications précédentes, où ledit ion marquant est choisi parmi des ions radioactifs de Tc, Re, Cu, Ag, Hg, Au et In.

19. Une méthode selon l'une quelconque des revendications précédentes, où la composition précédant l'étape (c) est conservée en flacons de dosage sous forme lyophilisée et réhydratée durant ou immédiatement avant l'étape (c).

20. Une composition pharmaceutique contenant un peptide marqué comme défini dans la revendication 1, utilisable pour l'administration à un patient, ledit peptide comprenant un domaine de fonction biologique (BD) et un domaine de liaison d'ion de métal marquant médicalement utile (MD) comprenant au moins un acide aminé contenant du soufre, azote et/ ou oxygène capable de complexer avec des ions d'un métal de transition choisi parmi Zn, Cu, Sn, Co et Ni, et un ion de métal marquant médicalement utile,
caractérisée en ce que:
(a) ledit MD est comme défini dans l'une quelconque des revendications 1 à 4, et
(b) ledit BD comprend une des séquences
Tyr - Ile - Gly - Ser - Arg (YIGSR) et
Ile - Lys - Val- Ala - Val (IKVAV)
(c) ledit ion de métal marquant est directement couplé audit soufre, azote et/ ou oxygène dudit MD.

21. Une composition selon la revendication 20, où le peptide contient une ou plusieurs séquences choisies parmi
Arg - Lys - Gln - Ala - Ala - Ser - Ile - Lys - Val - Ala - Val
(RKQAASIKVAV)
Cys-Ser-Arg-Ala-Arg-Lys-Gln-Ala-Ala-Ser-Ile-Lys-Val-Ala-Val-Ser-Ala-Asp-Arg
(CSRARKQAASIKVAVSADR) et
Cys - Asp - Pro - Gly - Tyr - Ile - Gly -Ser -Arg
(CDPGYIGSR).

22. Une composition selon la revendication 20 ou la revendication 21, où ledit ion de métal marquant est comme défini dans la revendication 17 ou la revendication 18.

23. Une composition selon l'une quelconque des revendications 20 à 22, où le peptide est présent dans un tampon aqueux comme défini dans l'une quelconque des revendications 11 à 16.

24. Une unité de dosage lyophilisée ou congelée contenant un agent de ciblage utilisable pour l'administration à un patient et prêt (après réhydratation) pour le marquage direct par l'addition de pertechnetate ou perhenate radioactif,
caractérisée en ce que ledit agent comprend un peptide comme défini selon la revendication 1 comprenant un domaine de fonction biologique (BD) et un domaine de liaison d'ion de métal marquant médicalement utile (MD), où
(a) ledit MD est comme défini selon l'une quelconque des revendications 2 à 4, et
(b) ledit BD comprend une des séquences de:
Tyr - Ile - Gly - Ser - Arg (YIGSR) et
Ile - Lys - Val- Ala - Val (IKVAV)

25. Une unité de dosage selon la revendication 24, où ledit peptide contient une ou plusieures séquences choisies parmi
Arg - Lys - Gln - Ala - Ala - Ser - Ile - Lys - Val - Ala - Val
(RKQAASIKVAV)
Cys-Ser-Arg-Ala-Arg-Lys-Gln-Ala-Ala-Ser-Ile-Lys-Val-Ala-Val-Ser-Ala-Asp-Arg
(CSRARKQAASIKVAVSADR) et
Cys - Asp - Pro - Gly - Tyr - Ile - Gly -Ser -Arg
(CDPGYIGSR).

26. Une unité de dosage selon la revendication 24 ou la revendication 25, où le peptide est présent dans un tampon aqueux comme défini selon l'une quelconque des revendications 11 à 16.

27. Une composition sous forme dosée contenant un agent de ciblage utilisable pour l'administration à un patient consistant en des contenus réhydratés d'une unité de dosage selon l'une quelconque des revendications 24 à 26, qui ont été marqués par l'addition d'un ion de métal marquant radioactif.

28. Une composition selon la revendication 27, où ledit ion de métal marquant est du pertechnetate radioactif.

29. L'utilisation d'un peptide comme revendiqué selon la revendication 5 ou la revendication 6 pour la préparation d'une composition selon l'une quelconque des revendications 20 à 23 ou 28 ou 29, ou d'une unité de dosage selon l'une quelconque des revendications 24 à 26 pour l'administration à un patient pour l'imagerie diagnostique des poumons, où ledit BD comprend la séquence
Ile - Lys - Val- Ala - Val (IKVAV).

30. L'utilisation selon la revendication 29, où le peptide contient la séquence
Arg - Lys - Gln - Ala - Ala - Ser - Ile - Lys - Val - Ala - Val
(RKQAASIKVAV) ou
Cys-Ser-Arg-Ala-Arg-Lys-Gln-Ala-Ala-Ser-Ile-Lys-Val-Ala-Val-Ser-Ala-Asp-Arg
(CSRARKQAASIKVAVSADR)

31. L'utilisation selon la revendication 30, où le peptide consiste essentiellement en la séquence
Cys-Ser-Arg-Ala-Arg-Lys-Gln-Ala-Ala-Ser-Ile-Lys-Val-Ala-Val-Ser-Ala-Asp-Arg
(CSRARKQAASIKVAVSADR)
incluant des répétitions de IKVAV.

32. L'utilisation d'un peptide selon la revendication 5 ou la revendication 6 pour la préparation d'une composition selon l'une quelconque des revendications 20 à 23 ou 27 ou 28, ou d'une unité de dosage selon l'une quelconque des revendications 24 à 26 pour l'administration à un patient pour la détection de sites d'accumulation de plaquettes ou de carcinomes, où ledit BD comprend la séquence
Tyr - Ile - Gly - Ser - Arg (YIGSR).

33. L'utilisation selon la revendication 32, où le peptide contient également la séquence
Asp - Pro - Gly.

34. L'utilisation selon la revendication 33, où le peptide consiste essentiellement en la séquence
Cys - Asp - Pro - Gly - Tyr - Ile - Gly -Ser -Arg
(CDPGYIGSR)
incluant des répétitions de YIGSR.

35. L'utilisation d'un peptide selon la revendication 1, contenant des groupes thiolates, complexés par les atomes de soufre des groupes thiolates avec des ions d'un métal de transition choisi parmi Zn, Cu, Sn, Co et Ni, pour le marquage direct par la réaction du peptide complexé dans un tampon pharmaceutiquement acceptable avec un ion de métal marquant médicalement utile ayant un ordre de liaison plus élevé que ledit ion de métal de transition, où lesdits ions de métal marquants remplacent lesdits ions de métal de transition complexés résultant en une composition comprenant un peptide marqué dans ledit tampon.

36. L'utilisation selon la revendication 35, où les ions de métal de transition sont des ions d'étain.
